# EUROPÄISCHE PATENTSCHRIFT

(11) **EP 1 824 505 B1**
(45) Veröffentlichungstag und Bekanntmachung des Hinweises auf die Patenterteilung: **25.02.2015**
(21) Anmeldenummer: 05850145.3
(22) Anmeldetag: 13.12.2005
(51) Int. Cl.: A61K 38/17, C07K 14/47

(54) **VERFAHREN ZUR BESTIMMUNG ENTZÜNDLICHER VORGÄNGE UND PHARMAZEUTISCHE ZUSAMMENSETZUNGEN ZUR BEHANDLUNG DERSELBEN**
METHOD FOR DETERMINING INFLAMMATORY PROCESSES, AND PHARMACEUTICAL COMPOSITIONS FOR THE TREATMENT THEREOF
PROCEDE POUR DETERMINER DES PROCESSUS INFLAMMATOIRES ET COMPOSITIONS PHARMACEUTIQUES POUR TRAITER CES PROCESSUS

(30) Priorität: 14.12.2004 DE 102004060385
(43) Veröffentlichungstag der Anmeldung: 29.08.2007
(73) Patentinhaber: Mertens, Peter René, 52072 Aachen (DE)
(72) Erfinder: Mertens, Peter René, 52072 Aachen (DE)
(74) Vertreter: Von Kreisler Selting Werner - Partnerschaft von Patentanwälten und Rechtsanwälten mbB
(86) Internationale Anmeldenummer: PCT/DE2005/002238
(87) Internationale Veröffentlichungsnummer: WO 2006/063567

(56) Entgegenhaltungen:
- DE-A1- 10 031 122
- BARGOU R C ET AL: "NUCLEAR LOCALIZATION AND INCREASED LEVELS OF TRANSCRIPTION FACTOR YB-1 IN PRIMARY HUMAN BREAST CANCERS ARE ASSOCIATED WITH INTRINSIC MDR1 GENE EXPRESSION" NATURE MEDICINE, NATURE PUBLISHING GROUP, NEW YORK, NY, US, Bd. 3, Nr. 4, April 1997 (1997-04), Seiten 447-450, XP001148855 ISSN: 1078-8956
- VAN ROEYEN CLAUDIA R C ET AL: "Y-box protein 1 mediates PDGF-B effects in mesangioproliferative glomerular disease" JOURNAL OF THE AMERICAN SOCIETY OF NEPHROLOGY, Bd. 16, Nr. 10, Oktober 2005 (2005-10), Seiten 2985-2996, XP009065141 ISSN: 1046-6673

## Beschreibung

Die vorliegende Erfindung betrifft ein Verfahren zur Untersuchung von extrazellulären Körperflüssigkeiten auf die Anwesenheit des Y-Box Proteins YB-1 und Fragmenten davon, die von der Zelle sezerniert worden sind, zur Bestimmung von entzündlichen Vorgängen und Tumorerkrankungen in Säugetieren. Die Erfindung umfasst des Weiteren die Verwendung von YB-1 als Marker.

### Beschreibung des Standes der Technik

Entzündungen sind vom Bindegewebe und den Blutgefäßen von Säugetieren getragene Reaktionen des Organismus auf einen äußeren oder innerlich ausgelösten Entzündungsreiz mit dem Zweck, diesen zu beseitigen oder zu inaktivieren und die reizbedingte Gewebsschädigung zu reparieren, was im Allgemeinen auch mit Wundheilung bezeichnet wird. Auslöser für solche Entzündungen können mechanische Reize, wie z.B. Druck, oder Fremdkörper, andere physikalische Faktoren, wie z.B. ionisierende Strahlung, UV-Licht, Temperatureinflüsse, aber auch chemische Stoffe, wie z.B. Säuren, Laugen, bakterielle Toxine, Allergene sowie Erreger wie z.B. Mikroorganismen, Würmer und Insekten, sein. Nicht nur die Vielfalt dieser Entzündungsreize, sondern auch die Vielfalt der damit verbundenen entzündlichen Vorgänge des Organismus macht eine genaue Diagnose dieser entzündlichen Vorgänge und seiner Ursachen erforderlich, um eine geeignete Therapie zur Bekämpfung des entzündlichen Vorganges und der damit verbundenen Erkrankung zu ermöglichen.

Zur Diagnose der zum jeweiligen entzündlichen Vorgang gehörigen Erkrankung bedient man sich verschiedenster Mittel. Zum Beispiel nutzt man neben bereits meist äußerlich erkennbaren Entzündungszeichen, wie z.B. Rötung der Haut, Wärmeentwicklung, Schmerzen und Geschwülste, die von außen ertastbar, sichtbar oder anders erkennbar sind, zur Diagnose auch die Ergebnisse von Untersuchungen verschiedener Marker in Körperflüssigkeiten, wie z.B. Blut und Urin. Diese Marker können z.B. niedermolekulare anorganische oder organische Substanzen, Proteine oder Zellen sein. Bei solchen Untersuchungen werden z.B. die im Blut oder Urin enthaltenen Markersubstanzen auf Unregelmäßigkeiten im Vergleich zu ihrer normalen Verteilung im Blut oder Urin untersucht. Die Ergebnisse können dann Rückschlüsse auf die Art und Ursache der Erkrankung ermöglichen. Als Beispiele für anhand des Blutbildes erkennbare Erkrankungen sind hier beispielhaft Leukämie oder das Pfeiffersche Drüsenfieber, eine Virusinfektion, genannt.

Es gibt aber auch entzündliche Erkrankungen, bei denen die Diagnose mittels äußerlich erkennbarer Entzündungszeichen und/oder der Bestimmung des Blutbildes oder des Urinstatus nicht ausreichend ist, um eine genaue Diagnose über die Art oder Ursache der Erkrankung zu erstellen. Als Beispiel sei die IgA-Nephropathie (IgAN auch als IgA-Nephritis bezeichnet) genannt, die eine Form der entzündlichen mesangioproliferativen Glomerulonephritis ist. Diese Erkrankung ist die häufigste Ursache für eine Niereninsuffizienz, die eine regelmäßige Dialyse für den Patienten erforderlich macht. Die für eine eindeutige Diagnose erforderlichen klinischen Leitsymptome können bei dieser Erkrankung sehr unterschiedlich sein. Als klinische Leitsymptome dienen zum Beispiel das Auftreten einer persistierenden Mikrohämaturie oder einer Makrohämaturie. Außerdem wird die Hämaturie häufig von einer Proteinurie, d.h. der Ausscheidung von Proteinen wie z.B. Albuminen, α₁-Globuline und β-Globuline, in größeren Mengen begleitet. Obwohl die klinischen Leitsymptome das Vorliegen einer solchen IgA-Nephritis sehr wahrscheinlich machen, ist zur letztendlichen Bestätigung der Diagnose eine Nierenbiopsie erforderlich, bei der eine Gewebeprobe der Niere für Laboruntersuchungen entnommen wird. Dieses sehr aufwendige Verfahren dient auch zur Bestimmung von prognostischen Parametern, die über den weiteren Verlauf der Erkrankung Auskunft geben können und die dem Arzt helfen, die Grundlage für eine geeignete Therapie zu schaffen (Friedrich Thaiss und Rolf A.K. Stahl, Deutsches Ärzteblatt, Jg.97, Heft 41, S.A2708-A2711, 13. Oktober 2000). Weitere entzündliche Erkrankungen, die eine Gewebebiopsie erforderlich machen sind z.B. eine Lupusnephritis, eine Vaskulitis oder eine membranöse Glomerulonephritis. Daher besteht weiterhin ein hoher Bedarf an Verfahren zur besseren Diagnose und Verlaufsbeurteilung von entzündlichen Vorgängen bzw. Erkrankungen, die den Einsatz von invasiven Methoden, wie der Biopsie, überflüssig machen.

Gleichartige Bestrebungen gibt es bei Tumorerkrankungen. Auch hier sind z.T. markerbasierende Nachweisverfahren für das Ausmaß der Tumorlast und das Metastasierungsverhalten der Tumoren etabliert worden, die das alpha-Fetoprotein für das hepatozelluläre Karzinoms oder das carcinoembryonale Antigen (CEA) und die Neuron-spezifische Enolase (NSE) für Bronchialkarzinome nachweisen. Ein Rezidiv eines Tumors kann teilweise durch den Anstieg eines Tumormarkers frühzeitig ermittelt werden, um anschließend diagnostische Untersuchungen zum Ausmaß des Tumors und der Lokalisation durchzuführen.

Im Stand der Technik wird daher intensiv sowohl nach extrazellulären als auch nach intrazellulären Markern gesucht, welche eine schnelle und eindeutige Diagnose von derartigen o.a. Erkrankungen ermöglichen. DE 100 31 122 A1 beschreibt zum Beispiel die Verwendung des Proteins YB-1, das für die Tumorprogression in den Zellen von Bedeutung ist, zur Diagnose von malignen Erkrankungen. Die Erfindung beruht unter anderem auf der Erkenntnis, dass das Protein YB-1 seine Funktion nur im Zellkern erfüllen kann.

YB-1 gehört zu einer Proteinfamilie, deren Vertreter an bestimmte einzel- oder auch doppelsträngige Nukleinsäureabschnitte, die auch als Y-Box bezeichnet werden, binden. Die Vertreter dieser Proteinfamilie treten sowohl bei Prokaryonten als auch bei Eukaryonten auf. Da bei ersten Untersuchungen an Prokaryonten festgestellt worden ist, dass diese Proteine als Antwort auf einen Kälteschock induziert werden (Goldstein, J., et al., Proceedings of the National Academy of Sciences USA, 1990, Vol.87, S.283-287), bezeichnete man sie als Kälteschockproteine ("*cold shock proteins*"). Die eukaryontischen YB-Proteine haben eine errechnete Masse von durchschnittlich 35 kDa und bestehen aus einem variablen Arginin und Alanin reichen N-Terminus, einer hochkonservierten Domäne von etwa 70 Aminosäuren und der abwechselnden Anordnung von jeweils 4 Clustern mit basischen/aromatischen bzw. sauren Aminosäuren im C-terminalen Bereich. Trotz ihrer niedriger berechneten Masse von durchschnittlich 35 kDa treten diese Proteine bei einer SDS-Page bei einem Molekulargewicht (MGW) von 50.000 bis 60.000 auf. Die starken Ladungsunterschiede im C-Terminalen Cluster sind vermutlich der Grund für das anomale Laufverhalten in der SDS-Page (Dissertation Karsten Jürchott HU-Berlin, November 1999, Titel: "Untersuchungen zur subzellulären Lokalisation und zu den Funktionen von YB-1, einem Y-Box-Protein in Säugerzellen"). Die für alle YB-Proteine charakteristische hochkonservierte Domäne ist die DNA-bindende Domäne, welche die Bezeichnung Kälteschockdomäne ("*cold shock domain"* CSD) erhielt. Die Aminosäure- als auch die Nukleinsäuresequenz des YB-1-Proteins wird zum Beispiel in dem Artikel von Didier et al. beschrieben (Proc. Natl. Acad. Sci. USA, 1988, Vol.85, S.7322-7326)

Die Klasse des YB-1-Proteins erfüllt innerhalb der Zelle vielfältige Aufgaben, die diese Proteine zu interessanten Targets machen. In dem Review-Artikel von S.K. Swamynathan et al. (The FASEB Journal, May 1998, Vol.12, P.515-522) werden die verschiedenen bisher bekannten Funktionen dieser Proteine innerhalb der Zelle beschrieben. Demnach spielen die YB-Proteine in vielen Zellen eine Rolle bei der Modulation der Transkription, bei der Modifikation des Chromatins, der Maskierung von RNA bei der Translation, im eukaryontischen Redox-Signalweg, bei der Regulation der Stressantwort der Zelle als auch bei der Aktivierung diverser Gene. Eine Beteiligung des YB-1 Proteins an extrazellulären Prozessen oder ein Auftreten des YB-1 Proteins außerhalb der Zelle war jedoch bis jetzt unbekannt.

### Detaillierte Beschreibung der vorliegenden Erfindung

Der Erfindung liegt die überraschende Entdeckung der Erfinder zu Grunde, dass das bisher nur als intrazellulärer Faktor bekannte Protein YB-1 bei entzündlichen Vorgängen und malignen Erkrankungen von Zellen nicht nur exprimiert wird, sondern auch von der Zelle ausgeschieden wird, somit von der Zelle in die extrazelluläre Flüssigkeit des Organismus sekretiert wird. Dies eröffnet die Möglichkeit für die Nutzung dieses Proteins als Marker für die Diagnose entzündlicher Vorgänge und maligner Erkrankungen.

Die Erfindung umfasst daher ein *in vitro* Verfahren zur Bestimmung eines entzündlichen Vorganges oder einer malignen Erkrankung in einem Säugetier, wobei die Bestimmung des entzündlichen Vorganges oder der malignen Erkrankung das Untersuchen einer Probe einer extrazellulären Flüssigkeit von einem Säugetier auf das Vorhandensein des YB-1 Proteins und/oder mindestens eines seiner Fragmente in der Probe umfasst.

Der Begriff "Bestimmung" eines entzündlichen Vorganges oder malignen Erkrankung umfasst dabei die Feststellung, ob ein entzündlicher Vorgang oder eine maligne Erkrankung im Organismus abläuft. In Bezug auf einen entzündlichen Vorgang bedeutet dies im speziellen, ob eine Reaktion des Organismus auf einen äußeren oder innerlich ausgelösten Entzündungsreiz vorliegt. Darüber hinaus umfasst diese Definition bei entzündlichen Erkrankungen auch die Bestimmung der Art des entzündlichen Vorganges anhand des Nachweises des YB-1 Volllängen-Proteins, Fragmenten des YB-1 Proteins oder beidem, die jeweils von den entsprechenden Zellen in die Körperflüssigkeit sekretiert worden sind. Des Weiteren lässt sich mit Hilfe des erfindungsgemäßen Verfahrens auch das Entzündungsstadium bestimmen, in dem sich der Körper des Säugetiers gerade befindet. In Bezug auf die malignen Erkrankungen lässt sich vor allem vorhersagen, ob ein metastasierender Tumor vorliegt oder nicht. Anstelle der Entnahme einer Gewebeprobe des potentiell tumorösen Gewebes erlaubt das erfindungsgemäße *in vitro* Verfahren eine schnelle und einfache Diagnose bei Patienten mit Verdacht auf einen bösartigen metastasierenden Tumor.

Das Verfahren der vorliegenden Erfindung eignet sich besonders zur Bestimmung von entzündlichen Vorgängen und malignen Erkrankungen in Säugetieren. Dabei sind alle Säugetiere gemeint, deren Körperzellen bei entzündlichen Vorgängen oder metastasierenden Tumorerkrankungen YB-1 und/oder Fragmente von YB-1 in extrazelluläre Körperflüssigkeiten sekretieren. Der Mensch ist dabei ein Beispiel für ein Säugetier, dessen Zellen bei entzündlichen Vorgängen oder malignen Erkrankungen YB-1 und Fragmente von YB-1 sekretieren. Beispielhaft wird dies in Beispiel 6 anhand der Sekretion des YB-1-Proteins durch isolierte primäre Makrophagen demonstriert. Weitere Zellarten, die YB-1 sezernieren, sind Mesangialzellen, B- und T-Lymphozyten, Tumorzellen, wie z.B. HepG2 Zellen, oder MonoMac-6 Zellen bei akuter Myelose.

Aufgrund der Tatsache, dass das in der vorliegenden Erfindung beschriebene YB-1 Protein und die Fragmente des YB-1 Proteins von der Zelle ausgeschieden werden, somit als sekretiertes YB-1 vorliegt, ist der Nachweis von YB-1, Proteinfragmenten von YB-1 oder Kombinationen davon in einer extrazellulären Körperflüssigkeit möglich. Die Entnahme von Körperflüssigkeit ist einfacher als zum Beispiel die Entnahme einer Gewebeprobe, die z.B. bei einer IgA-Nephritis zur entgültigen Sicherung der Diagnose erforderlich ist. Gleichzeitig ist mit dem Verfahren der vorliegenden Erfindung eine Frühdiagnostik einer Erkrankung möglich, ohne dass Gewebeproben entnommen werden und ohne dass bei Tumorerkrankungen eine Lokalisationsdiagnostik vorausgehen muss.

Als extrazelluläre Körperflüssigkeiten werden erfindungsgemäß Blut, Urin, Lymphe, Plasma, Serum, Schweiß, Nasensekret, Vaginalsekret, Wundexsudat, Sputum, Eiter, Samenflüssigkeit, Stuhl oder Liquor bezeichnet. Urin, Schweiß, Nasensekret, Vaginalsekret, Wundexsudat, Sputum, Eiter, Samenflüssigkeit und Stuhl können dabei einfach bei der Ausscheidung aus dem Körper aufgefangen oder durch einen Abstrich gewonnen werden. Blut, Plasma, Serum, Lymphe und Liquor können durch Nadelaspiration aus dem Gefäßsystem des Probanden gewonnen werden, wobei das Blut nach der Entnahme noch zentrifugiert werden muss, um Blutkörperchen-freies Blutplasma zu erhalten. Für den Anwender ist der Nachweis von YB-1 Proteinen, Fragmenten des YB-1 Proteins oder einer Kombination davon aus dem Urin die einfachste und schnellste Möglichkeit zur Bestimmung des entzündlichen Vorganges oder der malignen Erkrankung. Der Urin lässt sich dann auf YB-1 Proteine, Proteinfragmente des YB-1-Proteins oder Kombinationen davon kontinuierlich untersuchen.

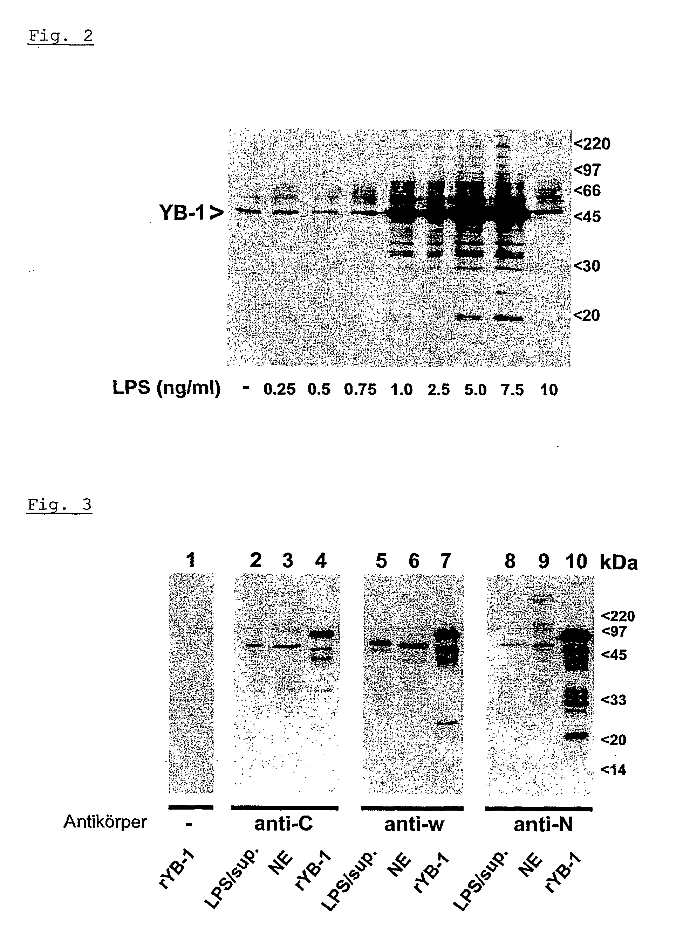

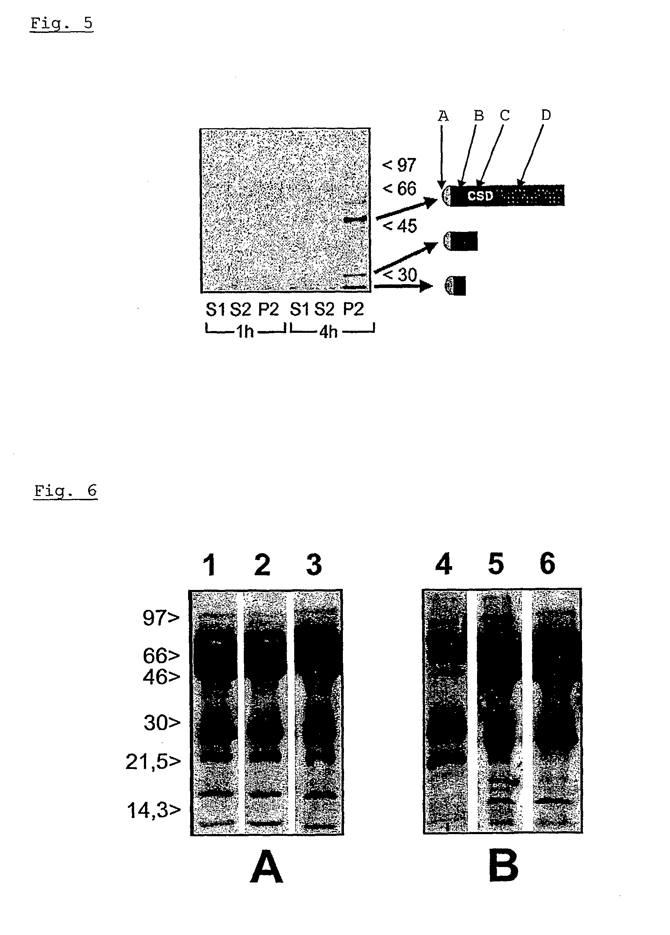

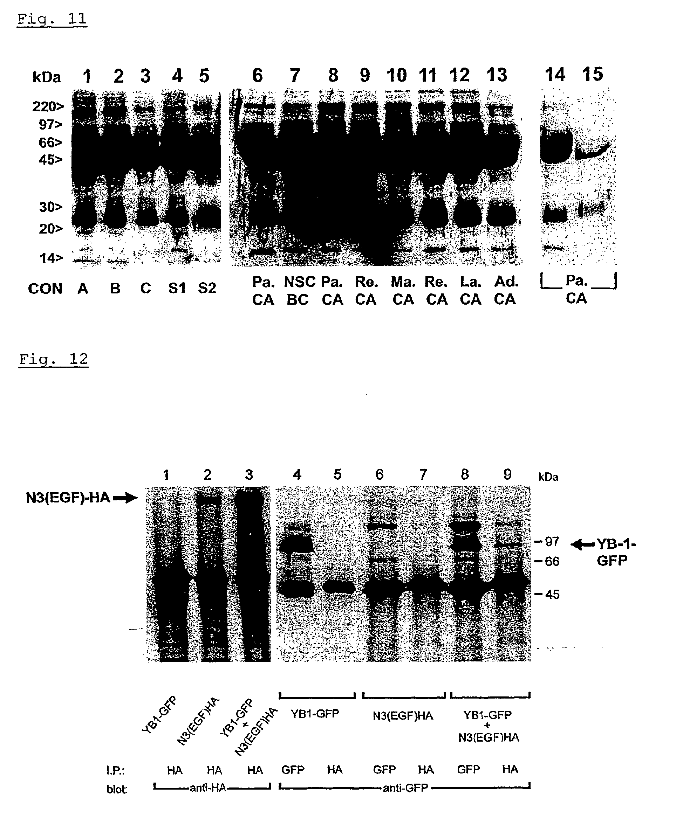

Das extrazellulär vorliegende YB-1 (Reihen "LPS/sup." in Fig.3) unterscheidet sich von nukleärem YB-1 (Reihen "NE" in Fig.3) durch eine geringfügig höhere Mobilität in einem Polyacrylamidgel, wie anhand der Fig. 3 (Beispiel 2) (mit verschiedenen, gegen unterschiedliche Epitope von YB-1 gerichteten Anti-YB-1 Antikörpern) zu sehen ist. Dieser Unterschied ergibt sich dadurch, dass das Protein modifiziert ist, wobei ein alternativer Sekretionsweg nachgewiesen wurde, d.h. YB-1 wird nicht über das ER oder den Golgi-Apparat der Zelle modifiziert. Zudem werden Fragmente des Proteins nachgewiesen, die sich entsprechend der Krankheitsaktivität ändern, wie z.B. bei der aktiven mesangioproliferativen IgA-Nephritis mit Nachweis von Fragmenten mit den Größen 28, 23, 16 und 8 kDa (siehe Fig. 6), bzw. bei Tumorerkrankungen im vermehrten Nachweis eines ungefähr 16 kDa großen Fragments im Serum (siehe Fig. 11).

Die vorliegende Erfindung macht sich beim Nachweis von sekretiertem YB-1 Protein durch die Zellen zu Nutze, dass das YB-1 Protein nach oder während der Sekretion aus der Zelle in kleinere Fragmente aufgespalten wird. Da im Rahmen von entzündlichen Nierenerkrankungen wie der IgA-Nephritis oder im Rahmen der Nierentransplantatabstoßung die kleineren Fragmente relativ krankheitsspezifisch gebildet werden, kann aufgrund des spezifischen Nachweises der Fragmente in einer Körperflüssigkeit, wie z.B. Urin, auf eine Krankheitsaktivität rückgeschlossen werden.

Das "Sekretionsmuster" ergibt sich aus dem unterschiedlichen Auftreten von YB-1, seiner Fragmente und Kombinationen davon. Neben dem Volllängen YB-1, dass nach einem Western-Blot bei 52 kDa auftritt, treten kleinere Fragmente mit Größen von 8 kDa, 16 kDa, 23 kDa, 28 kDa, 30 kDa, 32 kDa und 35 kDa auf. Je nach Verlauf eines entzündlichen Vorganges oder einer malignen Erkrankung kann sich die Verteilung und Menge der auftretenden Fragmente verändern. Weiterhin sind weitere YB-1 Fragmenten festzustellen, die bei noch nicht vollständig untersuchten Entzündungen bzw. malignen Erkrankungen auftreten können. Die Größe der Fragmente kann abhängig von der Durchführung der eingesetzten Nachweismethode geringfügig nach oben oder unten abweichen. Die Erfindung umfasst daher auch Fragmente, die ein Molekulargewicht aufweisen, das um bis zu ± 5 kDa von dem in dieser Erfindung in den Beispielen bestimmten Molekulargewicht abweicht. YB-1 und Fragmente von YB-1 lassen sich unter anderem mit Hilfe der in Tabelle 1 genannten Antikörper nachweisen.

Alternativ zum Nachweis des Volllängen YB-1 und der Fragmente von YB-1 mittels eines Western-Blots, so wie er in Beispiel 1 beschrieben wird, kann die Bestimmung des Sekretionsmusters auch über HPLC erfolgen. Im Vergleich zum Western-Blot ermöglicht HPLC eine bessere Quantifizierung der detektierten Fragmente. Weitere Einzelheiten zur HPLC und anderen Verfahren der Proteinanalytik finden sich im Buch "Bioanalytik" von F. Lottspeich und H. Zorbas (Spektrum Akademischer Verlag, Mai 1998, 1. Auflage, ISBN 3-8274-0041-4). Andere Methoden die in der Proteinanalytik verwendet werden, sind dem Durchschnittsfachmann bekannt. Beispielhaft genannt sind Färbetests, wie das Biuret-Assay, das Lowry-Assay, das Bicinchoninsäure-Assay (BCA-Assay), das Bradford-Assay, spektroskopische Methoden zu denen z.B. neben der UV-und Fluoreszenzmessung auch die radioaktive Markierung, die Iodierung von Proteinen und die Massenspektrometrie gehören, enzymatische Tests und immunologische Nachweistechniken, chromatographische Trennmethoden, elektrophoretische Verfahren, die Kapillarelektrophorese, die Sequenzanalyse und Röntgenstrukturanalyse.

Ein YB-1 Sekretionsmuster mit Auftreten von Fragmenten der Größe 8, 16 und 23 kDa im Urin wird zum Beispiel bislang ausschließlich bei Erkrankungen beobachtet, die mit einer Entzündung der Mesangialzellen in den Nierenkörperchen einhergehen. Im Rahmen von Nierentransplantat-Abstoßungen ist immunhistochemisch nachgewiesen worden, dass die charakteristischen Zellinfiltrate aus Monozyten und Lymphozyten erhebliche Mengen an YB-1 exprimieren. Aufgrund des Sekretionsmusters im Urin und der Befunde mit MM-6 Zellen nach ATP und LPS-Stimulation kann davon ausgegangen werden, dass Entzündungszellen YB-1 und dessen Fragmente bei der Abstoßungsreaktion sezernieren und sie daher im Urin nachweisbar sind.

Volllängen-YB-1 sowie ein 30 kDa YB-1 Fragment sind in erheblicher Menge im Serum gesunder Probanden nachweisbar. Untersucht man das Serum von Patienten mit verschiedenen metastasierenden Tumorerkrankungen im Western-Blot mit einem gegen das Volllängen-YB-1 Protein gerichteten polyklonalen Antikörper sowie mit einem monoklonalen Antikörper, so ist zusätzlich bei allen untersuchten Tumorpatienten (n=15) eine 16 kDa große YB-1 Fragment-Bande nachweisbar (Fig. 11). Das 16 kDa Fragment ist bei den getesteten drei gesunden Probanden und dem Sepsispatienten S2 nicht nachweisbar. Entsprechende Befunde wurden mit verschiedenen polyklonalen Antikörpern gegen Volllängen-YB-1 erhoben.

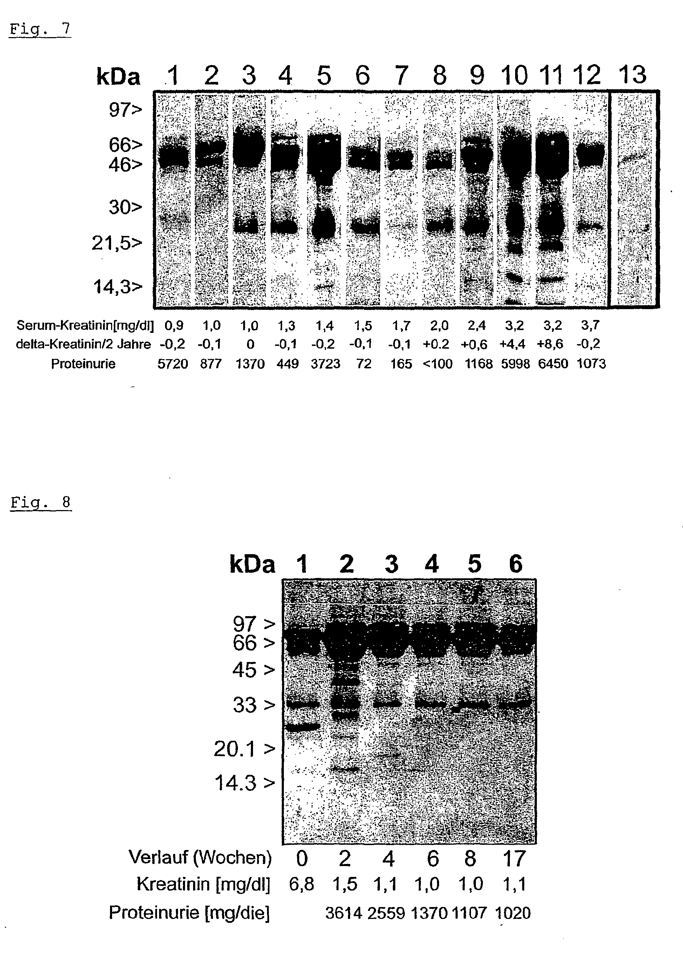

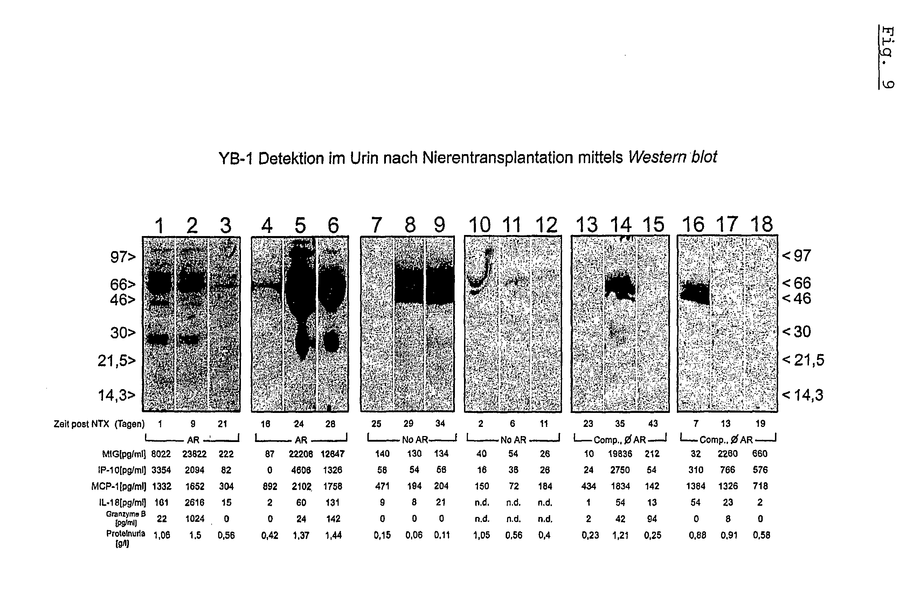

Daher umfasst das Verfahren der vorliegenden Erfindung außerdem das Auswerten der bei der obigen Untersuchung erhaltenen Untersuchungsergebnisse zur Bestimmung eines Sekretionsmusters des YB-1 Proteins und seiner Fragmente in der Probe. Die Menge und Größe der sich bei der Aufspaltung ergebenden einzelnen Proteinfragmente von extrazellulärem YB-1 unterscheiden sich, je nach entzündlichem Vorgang und je nach Stadium, in dem sich dieser entzündliche Vorgang befindet, deutlich von der Größenverteilung der einzelnen Proteinfragmente des YB-1, die bei anderen entzündlichen Vorgängen entstehen. So unterscheidet sich das "Sekretionsmuster" von YB-1 und dessen Proteinfragmente bei einer Abstoßungsreaktion nach einer Nierentransplantation, wie in Fig. 9 (Beispiel 5) zu sehen, deutlich von dem YB-1 Sekretionsmuster bei einer IgA-Nephritis, dargestellt in Fig. 6 und 7 (Beispiel 4). Das bedeutet, dass der Anwender des erfindungsgemäßen Verfahrens anhand der Verteilung des YB-1 und der YB-1 Proteinfragmente Art, Ursache und Stadium, in dem sich die entzündliche Erkrankung befindet, bestimmen kann. Daher umfasst das Verfahren der vorliegenden Erfindung des Weiteren das Vergleichen des zuvor bestimmten Sekretionsmusters mit anhand von Eichreihen für entzündliche Vorgänge erstellten Sekretionsmustern. Dabei umfasst das dabei bestimmte Sekretionsmuster immer auch das Volllängen YB-1 Protein selbst. Die Eichreihen lassen sich anhand von Mehrfachmessungen bei unterschiedlichen Patienten ermitteln, so wie es exemplarisch in den Beispielen 4, 5 und 7 geschildert wird.

In Abbildung 8 ist bei einem Patienten mit IgA-Nephritis anhand der YB-1-Urindiagnostik erkenntlich, dass die Nierenfunktion sich im Anschluss an das Verschwinden der niedermolekularen Banden über einen zeitlichen Verlauf normalisiert. Aufgrund des Nachweises der niedermolekularen Bande kann z.B. eine Entscheidung für die Behandlung zur Immunsuppression abgeleitet werden. Zudem ermöglicht der Nachweis des 16 kDa Fragments im Serum die Diagnostik bzw. Verlaufsbeurteilung von Tumorerkrankungen (Fig. 11, Beispiel 7).

Die in Fig. 6 und 7 dargestellten Ergebnisse machen deutlich, dass das Sekretionsmuster bei unterschiedlichen Patienten nahezu identisch auftritt. Fig. 6 zeigt die Untersuchungsergebnisse zweier Patienten, die beide einen progredienten Verlauf der IgA-Nephritis aufweisen. Dabei zeichnet sich das YB-1 Sekretionsmuster für diese Patienten neben dem Volllängen YB-1 bei 52 kDa auch durch Fragmente bei 28, 23, 16 und 8 kDa im Gel aus. An dieser Stelle ist angemerkt, dass sich die im Rahmen der Beschreibung der Erfindung angegebenen kDa-Werte auf die Lage der Banden im Gel beziehen. In Fig. 7 ist der Urinuntersuchungsbefund auf YB-1 mit Spontanurinen von 12 Patienten mit bioptisch gesicherter IgA-Nephritis durchgeführt worden. Die Patientendaten werden entsprechend der Höhe des Serumkreatinins beim Auftragen im Gel sortiert und der Verlauf der Kreatininkonzentration in den folgenden 2 Jahren angegeben. Ein Sekretionsmuster, in dem neben dem Volllängen YB-1-Protein und dem 28 kDa Fragment noch kleinere Fragmente mit einer Größe von 23, 16 und 8 kDa auftreten (Spuren 10 und 11) gehen mit einer Progredienz der Erkrankung bis hin zur terminalen, dialysepflichtigen Niereninsuffizienz einher. Die durch solche Messungen anhand von Patienten mit bekanntem Krankheitsverlauf ermittelten Sekretionsmuster für YB-1 können daher als Eichreihe für die Bestimmung dieser entzündlichen Vorgänge bei anderen Patienten verwendet werden.

Wie anhand der Ergebnisse in Fig. 9 (Beispiel 6) weiter zu erkennen ist, ist der Nachweis des YB-1 Proteins und seiner Fragmente als Markersubstanz für den Nachweis von entzündlichen Vorgängen, in diesem Beispiel einer Organabstoßung, deutlich früher möglich als der Nachweis mit herkömmlich verwendeten Markern. Wie anhand von Fig. 9 zu erkennen ist, ist beim Patienten A das kleinere YB-1 Proteinfragment bei 26 kDa bereits vor dem Anstieg der Konzentration des für die Abstoßungsreaktion als Frühmarker eingesetzten Granzym B oder MIG zu erkennen. Der hierbei verwendete monoklonale Antikörper (AB 4) erkennt das genannte Fragment.

Die Ergebnisse der durchgeführten Versuche erlauben außerdem die Schlussfolgerung, dass die Anwendung des Verfahrens der vorliegenden Erfindung bei entzündlichen Vorgängen in der Niere, wie z.B. bei einer IgA-Nephritis, unabhängig vom Ausmaß einer Proteinurie ist, die normalerweise bei Nierenerkrankungen zur genaueren Diagnose des entzündlichen Vorganges herangezogen wird. Die vorliegende Erfindung stellt damit ein wirksames Frühwarnsystem für die Diagnose von entzündlichen Vorgängen und malignen Erkrankungen im Säugetierorganismus dar.

Der Nachweis des extrazellulär auftretenden YB-1 Proteins, des mindestens einen YB-1 Proteinfragmentes oder Kombinationen davon erfolgt mittels im Stand der Technik bekannter Verfahren. Dazu gehören z.B. die Proteinbestimmung mittels Färbetests, enzymatischer Aktivitätstests, immunologischer und spektroskopischer Verfahren. Eine Übersicht zu diesen Techniken findet sich z.B. im Buch "Bioanalytik" von F. Lottspeich und H. Zorbas (Spektrum Akademischer Verlag, Mai 1998, 1. Auflage, ISBN 3-8274-0041-4). Für den Nachweis des YB-Proteins eignet sich zum Beispiel der immunologische Nachweis mit Antikörpern. Nach der Auftrennung der z.B. im Urin enthaltenen Proteine in einem Western-Blot werden polyklonale Antikörper oder monoklonale Antikörper verwendet, die gegen unterschiedliche Abschnitte des Volllängen YB-1 Proteins gerichtet sind und die daher auch zur Detektion von Proteinfragmenten von YB-1 verwendet werden können. Der Nachweis von YB-1 und Fragmente davon mittels Antikörper ist von Vorteil, da dieser immunologische Nachweis sehr spezifisch und sehr empfindlich ist. Im Stand der Technik z.B. in DE 100 31 122 A1 werden Antikörper beschrieben, die gegen das intrazelluläre YB-1 Protein gerichtet sind, das erst nach dem Aufschluss der Zellen aus den Gewebeproben für immunologische Untersuchungen zugänglich wird.

Das Verfahren der vorliegenden Erfindung eignet sich besonders zur Bestimmung von entzündlichen Vorgängen im Säugetierorganismus'. Ein solcher entzündlicher Vorgang kann dabei eine Abstoßungsreaktion nach einer Transplantation oder eine entzündliche Erkrankung sein. Die entzündliche Erkrankung wird dabei aus der Gruppe ausgewählt, die aus nephrologischen Erkrankungen, Asthma, chronisch obstruktiven Lungenerkrankungen, rheumatoider Arthritis, Vaskulitis, Diabetes mellitus, Krebs, Leukämien, Sepsis, Pankreatitis, Multipler Sklerose, Psoriasis, und Dermatitis besteht. Jeder Krankheit wird dabei je nach Krankheitsstadium ein spezifisches Sekretionsmuster für das Protein YB-1, YB-1 Proteinfragmenten oder Kombinationen davon zugeordnet. Das Verfahren eignet sich besonders zur Bestimmung nephrologischer Erkrankungen.

Die nephrologische Erkrankung wird dabei aus der Gruppe ausgewählt, die aus Glomerulonephritis, insbesondere IgA-Nephritis, interstitieller Nephritis, Pyelonephritis, lupoider Nephritis, radiogener Nephritis, vaskulärer Nephropathie und Zystennieren besteht. Beispiele für unterschiedliche Sekretionsmuster bei unterschiedlichen Erkrankungen (z.B. IgA-Nephritis und Organabstoßung) bzw. unterschiedlichen Stadien der Erkrankung, wie z.B. bei der in den folgenden Beispielen beispielhaft diskutierten IgA-Nephritis, werden anhand der Beispiele ersichtlich.

Wie in den Beispielen gezeigt wird, eignet sich das Verfahren der vorliegenden Erfindung auch zur Bestimmung von malignen Erkrankungen. Die malignen Erkrankungen umfassen dabei Tumore, die aus der Gruppe ausgewählt werden, die aus soliden Tumoren wie z.B. einem Pankreas-Karzinom, Rektum-Karzinom, Magen-Karzinom, Colon-Karzinom, Mamma-Karzinom, Malignes Melanom, Nierenzell-Karzinom, Larynx-Karzinom, Ovarial-Karzinom, Prostata-Karzinom, Bronchial-Karzinom und Leukämien, wie akuter myeloischer, akuter lymphozytärer, chronisch-myeloischer und chronisch-lymphatischer Leukämie besteht.

Die Erfindung umfasst weiterhin die Verwendung von YB-1 oder mindestens eines Fragmentes davon oder deren Kombination zur Bestimmung eines entzündlichen Vorganges in einer extrazellulären Flüssigkeit bei einem Säugetier. Dadurch dass die Sekretion von YB-1 bei entzündlichen Vorgängen und malignen Erkrankungen aller Art zu beobachten ist, eignet sich YB-1 oder mindestens eines seiner Fragmente oder deren Kombination besonders gut als Marker für entzündliche und maligne Erkrankungen im Körper eines Säugetiers. Dabei eignen sich für den Nachweis des YB-1 Proteins, mindestens einer seiner Fragmente oder Kombinationen davon besonders immunologische Techniken, die sich die spezifische Antikörper/Antigen-Bindung zu Nutze machen.

Für den Nachweis des YB-1 Proteins, mindestens einer seiner Fragmente oder Kombinationen davon ist ein Detektionskit geeignet, das zur Bestimmung eines entzündlichen Vorganges in einem Säugetier dient. Das Detektionskit umfasst dabei zumindest einen Antikörper, der zum Nachweis des YB-1 Proteins und/oder mindestens eines Fragmentes davon geeignet ist. Mittels des Detektionskits wird das Sekretionsmuster für YB-1 ermittelt und mit den bereits vorhandenen Eichreihen für entzündliche Vorgänge oder maligne Erkrankungen verglichen. Die frühzeitige Diagnose und Bestimmung des entzündlichen Vorganges oder der malignen Erkrankung ermöglicht es, frühzeitig eine geeignete Therapie für den Patienten zu entwickeln.

Die Mittel zum Nachweis des Proteins YB-1 umfassen Antikörper, wobei diese Antikörper sowohl monoklonale als auch polyklonale Antikörper sein können, die an mindestens eine antigenische Determinante des YB-1 Proteins oder Fragmente davon spezifisch binden können. Für das Verfahren der vorliegenden Erfindung geeignete Antikörper sind in Tabelle 1 aufgeführt. Die Antikörper beinhalten, sind dabei jedoch nicht begrenzt auf IgG, IgA, IgE, IgM und IgD-Antikörper. Diese Mittel können sowohl Puffer für die Aufbewahrung der Antikörper enthalten als auch Reagenzien, welche die Antikörper vor einem proteolytischen Abbau schützen.

Die Erfinder haben herausgefunden, dass das sezernierte YB-1 durch para- und autokrine Wirkung Entzündungsprozesse sowie die Zellproliferation beeinflusst. So konnte von den Erfindern in mehreren Experimenten gezeigt werden, dass YB-1 an Rezeptoren aus der NOTCH-Familie bindet. Die NOTCH-Rezeptoren sind eine Familie von Transmembranrezeptoren, die an der Regulation vieler Differenzierungsprozesse in der Zelle beteiligt sind, wie bei der Differenzierung von Stammzellpopulationen während der Muskelentwicklung oder Hämatopoese. Außerdem ist eine anti-apoptotische Wirkungsweise der aktivierten, humanen Variante des NOTCH-1-Rezeptors in T-Zellen bekannt, wodurch die Entwicklung bestimmter Zellpopulationen reguliert wird. Es sind mehrere Verbindungen zwischen NOTCH- und NF-κB-abhängigen Signalwegen bei der Aktivierung immunkompetenter Zellen unter dem Einfluss proinflammatorischer Stimuli (TNF-α-bakterielle Lipopolysaccharide) festgestellt worden. Durch Applikation von rekombinantem YB-1 kann die Aktivität von YB-1 gesteigert werden und eine Zelldifferenzierung eingeleitet werden, die zum Beispiel bei proliferativen Erkrankungen wie Neurodermitis von Bedeutung ist. Dadurch kann die Proliferation einer Zelle gehemmt und eine Differenzierung erreicht werden. Im Gegensatz dazu kann durch die Applikation von Antikörpern, die sezerniertes YB-1 binden, die Rekrutierung von Entzündungszellen (einwandernde Monozyten/Makrophagen) gehemmt werden. Wünschenswert ist dies zum Beispiel zur Vermeidung von Abstoßungsreaktionen bei Transplantaten aber auch bei der Prävention einer Atherosklerose oder Intimahyperplasie nach Koronarintervention und Stenteinlage. Weitere Anwendungen liegen in der Behandlung von Tumorerkrankungen, bei denen eine Differenzierung der Zellen zu einer Wachstumshemmung führt.

So konnte von den Erfindern in einem Versuch mit einer Keratinozytenkultur (Beispiel 8) gezeigt werden, dass YB-1 und Antikörper gegen YB-1 die Differenzierung der Zellen beeinflussen. Des Weiteren konnte gezeigt werden, dass YB-1 an TGF-β-binding-protein (LTBP) bindet und dadurch die Freisetzung weiterer Entzündungsmediatoren, wie z.B. TGF-β, bewirkt.

Die monoklonalen Antikörper, die in der vorliegenden Erfindung verwendet werden, werden aus der Gruppe von monoklonalen Antikörpern ausgewählt, die von Hybridoma-Zelllinien hergestellt werden, die in Tabelle 1 dargestellt sind. Bevorzugt ist ein monoklonaler Antikörper der Hybridoma-Zelllinie mit der Hinterlegungsnummer DSM ACC 2703.

### Beschreibung der Figuren

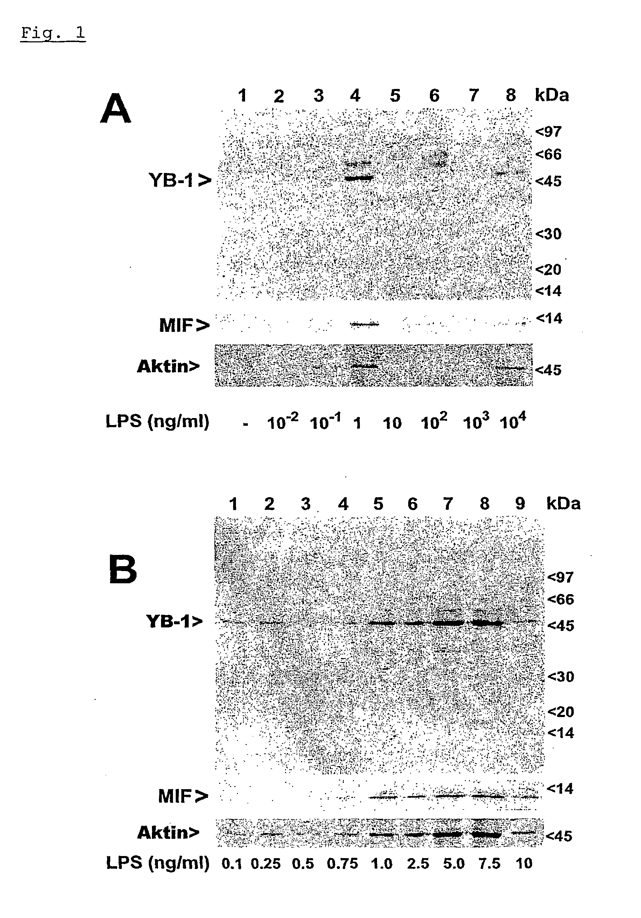

**Fig. 1A** und **1B** zeigen den Nachweis von YB-1 und seiner Fragmente mittels polyklonaler Antikörper, die gegen den C-Terminus des Proteins gerichtet sind (Mertens P.R., Alfonso-Jaume M.A. et al., J. Am. Soc. Nephrology, 1999, Vol.10, S.2480-2487) nach Induktion von monozytären MM-6-Zellen mit Lipopolysaccharid-(LPS)Lösungen unterschiedlicher Konzentration. In Fig. 1A erfolgte die Induktion der YB-1 (Bande bei 52 kDa) Sekretion durch die monozytäre Zelllinie MM-6 bei einer LPS-Konzentrationen von 1.0 ng/ml in Spur 4 (Reihe 1 dient als Kontrolle, keine Zugabe von LPS (-)). Niedrigere (10⁻² bis 10⁻¹ ng/ml) und höhere Konzentrationen (10 bis 10⁴ ng/ml) von LPS führten nicht, bzw. nur zu einer geringen Sekretion. Bei der gleichen Konzentration von LPS, 1 ng/ml, wurde eine Sekretion des *macrophage migratory inhibitory factor* (MIF) beobachtet und Aktin war ebenfalls nachweisbar. Dies belegt die Spezifität des Sekretionsmechanismus und dass es sich nicht um eine Zelllyse handelt. In Fig. 1B wurde der LPS-Konzentrationsbereich zwischen 0,25 und 10 ng/ml mit MM-6-Zellen ausgetestet. Es zeigt sich, dass YB-1 bei einer LPS-Konzentration von 1,0 bis 7,5 ng/ml sezerniert wird und mit dem polyklonalen, gegen den C-Terminus des Proteins generierten Peptidantikörper nachweisbar ist.
**Fig. 2** zeigt ähnlich wie in Fig. 1 die Ergebnisse eines Western-Blots mit längerer Antikörper-Exposition. So ist eine eine konstitutive Sekretion von YB-1 auch bei niedrigeren LPS-Konzentrationen (0,25 bis 0,75 ng/ml) nachweisbar. Zudem wird bei LPS-Konzentrationen von 1 bis 7,5 ng/ml eine Sekretion von YB-1 Spaltprodukten mit molekularen Massen von 35, 32, 28 und 16 kDa beobachtet.
**Fig. 3** zeigt die Unterschiede im Molekulargewicht zwischen rekombinant in *E*. *coli* hergestelltem YB-1 (rYB-1), nukleärem YB-1 (NE) und nach LPS-Stimulation von MM-6-Zellen sekretiertem YB-1 (LPS/sup.) in einem Western-Blot. Die dabei verwendeten Antikörper richten sich zum einen gegen den C-Terminus (anti-C) den N-Terminus (anti-N) und das Volllängen YB-1 Protein (anti-w). Die erste Reihe im Gel dient der Negativkontrolle (d.h. es wurde kein Erst-Antikörper verwendet). Nähere Einzelheiten zu diesem Versuch sind in Beispiel 2 zu finden. Es zeigt sich eine geringere Mobilität von sekretiertem YB-1 (Spuren 1, 5 und 8) im Vergleich zu YB-1 nukleären Ursprungs (Spuren 3, 6 und 9). Das prokaryontisch hergestellte YB-1 (rYB-1) unterscheidet sich nicht nur in seinem Molekulargewicht von 46 kDa vom eukaryontisch hergestellten YB-1 (LPS/sup.), es ist zudem als höhermolekulare Bande (88 kDa) nachweisbar. Die höhermolekulare Bande ist am ehesten aufgrund von Homomultimerisierung zu erklären. Zudem können Fragmente mit einer Größe von 38, 36, 35, 33 und 22 kDa nachgewiesen werden. Mit dem gegen das Volllängen-YB-1 Protein hergestellten Antikörper ist bei rekombinantem YB-1 zudem ein 25 kDa Fragment nachweisbar (Spur 7).

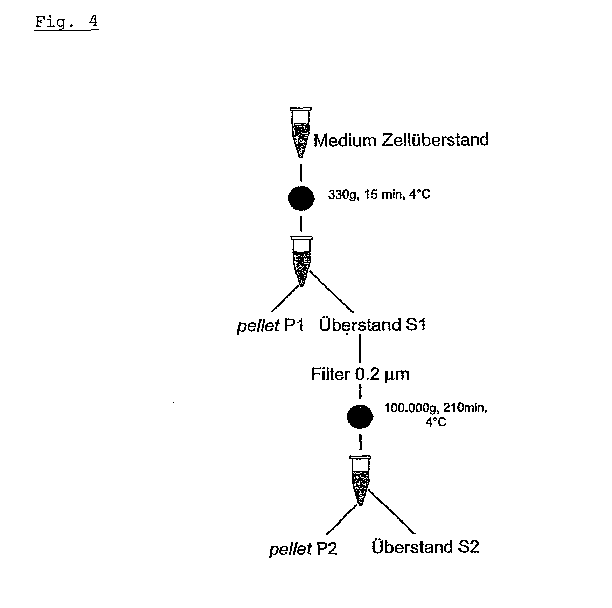

**Fig. 4** zeigt eine schematische Darstellung des Verfahrens zur Isolation von YB-1 und seiner Fragmente aus den YB-1 enthaltenden Mikrovesikeln, die von den mit PDGF-B induzierten Mesangialzellen sekretiert worden sind. Nähere Einzelheiten zum Versuch sind im Beispiel 3 zu finden. P1 steht dabei für Sediment 1, dass nach der ersten Zentrifugation des Zellüberstandes nach 1h bzw. 4h nach Zytokin-Inkubation gewonnen wird und das hauptsächlich Zellen, hochmolekulare Bestandteile und Zellfragmente enthält. S1 steht für den Zellüberstand, der nach der ersten Zentrifugation verbleibt und anschließend zentrifugiert wird, um Mikrovesikel zu gewinnen. S2 steht für den hierbei gewonnenen Überstand und P2 für das zweite Sediment, welches das YB-1 Protein und seine Fragmente in Mikrovesikeln enthält.
**Fig. 5** stellt die Ergebnisse des immunologischen Nachweises des sekretierten YB-1 von Mesangialzellen in einem Western-Blot dar. Die Bedeutung der Abkürzungen S1, S2 und P2 finden sich in der Beschreibung zu Fig. 4. Die mit dem Buchstaben "A" markierte Domäne ist die N-Terminal Domäne von sekretiertem YB-1. "B" steht für die Arginin-Alanin-reiche Domäne des sekretierten YB-1, "C" steht für die *cold-shock* Domäne (CSD) des sekretierten YB-1 und "D" steht für die alternierend positiv und negativ geladenen Abschnitte am C-Terminus von sekretiertem YB-1. In diesem Versuch wurde ein mit einem Hämagglutinin-Tag versehenes YB-1 Protein, das durch die Rattenmesangialzellen exprimiert wird, im Western-Blot mit einem Anti-HA Antikörper nachgewiesen. Dieses Vorgehen schließt aus, dass es sich bei dem sezernierten Protein um ein anderes Mitglied der Kälteschockproteinfamilie handelt, zu der YB-1 gehört. Nähere Einzelheiten zum Versuch siehe Beispiel 3.
**Fig. 6** zeigt den mit einem polyklonalen, gegen das ganze YB-1 Protein gerichteten Antikörper geführten immunologischen Nachweis des Proteins YB-1 (Bande bei 52 kDa) und seiner proteolytischen Fragmente mit einer Größe von 28, 23, 16 und 8 kDa, die aus dem Urin von Patienten gewonnen worden sind. In Fig. 6 sind jeweils 3 Proben von zwei Patienten (A und B) mit IgA-Nephritis und einer Proteinurie von über 1,5 g/d aufgetragen, die in mehrwöchigen Abständen gewonnen wurden. Sie alle zeigen ein nahezu identisches Sekretionsmuster von YB-1 und proteolytischen Fragmenten von YB-1. Nähere Einzelheiten zu den Ergebnissen finden sich im Beispiel 4.
**Fig. 7** zeigt ein Gel von zwölf Urinproben, , die von Patienten mit IgA-Nephritis gewonnen und die nach aufsteigendem Kreatinin-Wert sortiert wurden. Bei dem immunologischen Nachweis mit einem gegen das Volllängen-YB-1 Protein gerichteten Antikörper fällt auf, dass neben dem Volllängen-YB-1 Protein bei 52 kDa auch Fragmente mit den Molekulargewichten von 28, 23, 16 und 8 kDa nachweisbar sind. Nach Probenasservierung wurden die Patienten bezüglich ihres klinischen Verlaufs evaluiert und der Anstieg des Serumkreatinins angegeben. Während die Nierenfunktion bei den meisten Patienten stabil war, kam es bei den beiden Patienten mit Nachweis der 23, 16 und 8 kDa YB-1 Fragmente zu einer Progression hin zum terminalen Nierenversagen und Dialysepflichtigkeit. Hierbei fällt zudem auf, dass der Nachweis von YB-1 unabhängig vom Ausmaß der Proteinurie erfolgt.
**Fig. 8** zeigt exemplarisch den Verlauf bei einem Patienten mit bioptisch gesicherter IgA-Nephritis, bei dem sich das YB-1 Sekretionsmuster im Urin im Verlauf mehrerer Wochen änderte. Hierbei fällt auf, dass die kleinmolekularen Banden verschwinden und es zu einer Restitutio der Nierenfunktion kommt. Der Patient wurde zum Zeitpunkt des akuten Nierenversagens (Zeitpunkt 0) immunsuppressiv behandelt.
**Fig. 9** zeigt den immunologischen Nachweis von sekretiertem YB-1 und seiner Proteinfragmente bei einer akuten Nierentransplantatabstoßungreaktion aus dem Urin von 6 Patienten mittels eines monoklonalen, gegen YB-1 gerichteten Antikörpers. Die Urinproben wurden sequenziell nach erfolgter Nierentransplantation bei den untersuchten 6 Patienten (A-F) gesammelt. Bei den in Fig. 9 mit AR bezeichneten Patienten A und B liegt eine durch Biopsie gesicherte Abstoßungsreaktion (AR) vor. Bei den Patienten C und D liegen weder eine Abstoßungsreaktion noch irgendwelche weiteren Komplikationen vor (No AR). Bei den Patienten E und F traten zwar keine Abstoßungsreaktionen auf, jedoch gab es andere, infektiöse Komplikationen (comp., Ø AR). Parallel zur Bestimmung der Anwesenheit von YB-1 und dessen Fragmente wurden auch Cytokine und andere Markerproteine für Abstoßungsreaktionen bestimmt (MIG in pg/ml; IP-10 in pg/ml; MCP-1 in pg/ml; IL-18 in pg/ml; Granzym B in pg/ml; Proteinurie in g/l).

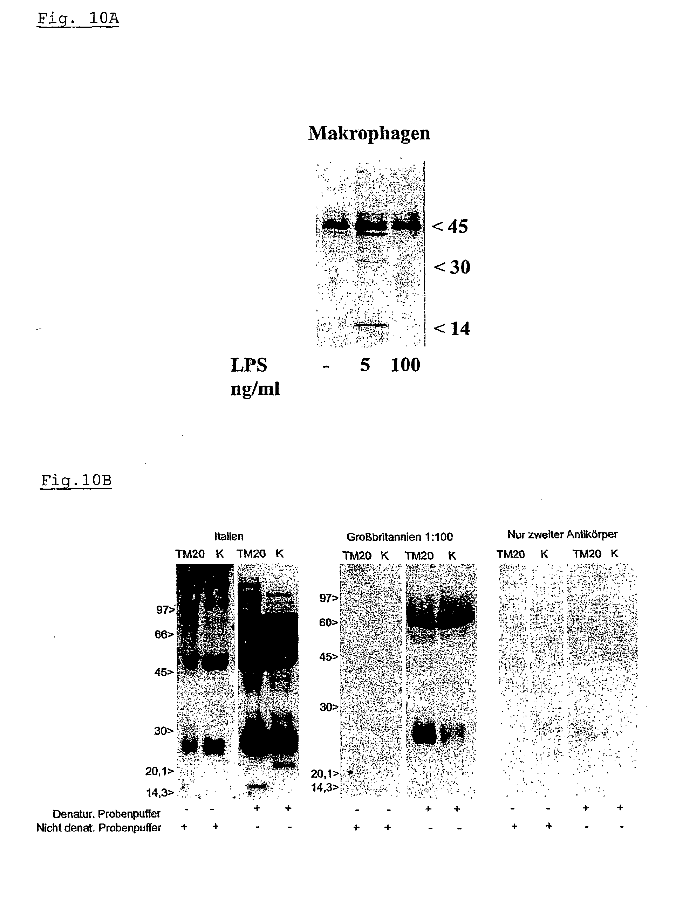

**Fig. 10A** zeigt die Ergebnisse eines Versuchs in dem primäre humane Monozyten und Makrophagen aus dem Blut eines gesunden Probanden isoliert und mit LPS inkubiert wurden. Nach Stimulation mit LPS in einer Konzentration von 5 ng/ml war Volllängen-YB-1 sowie ein 16 kDa Fragment im Serum-freien Überstand nachweisbar.
**Fig. 10B****.** zeigt den Nachweis von "spezifischen" YB-1 Fragmenten im Serum von Tumorpatienten und im Urin bei IgA-Nephritis mit einem monoklonalen Antikörper. Weitere Einzelheiten dazu siehe Ausführungsbeispiel 9. Mit dem generierten monoklonalen Antikörper "Italien" (AB8, siehe Tabelle 1) wurden das Serum eines Tumorpatienten mit metastasierendem Rektum-Karzinom (TM20) sowie Urin von einem Patienten mit progredienter IgA-Nephritis (K) untersucht. Hierbei fallen neben hochmolekularen Banden um 200 kDa auch Banden mit relativen Molekulargewichten von 52 und 28 kDa im nicht denaturierenden Probenpuffer (ohne Mercaptoethanol und EDTA) auf. Unter denaturierenden Bedingungen (mit Mercaptoethanol und EDTA) sind die zuvor mit verschiedenen polyklonalen Anti-YB-1 Antikörpern gesehenen Banden mit den relativen Größen von 16 kDa im Serum bei Tumorerkrankung bzw. 23 kDa bei progredienter IgA-Nephritis im Urin nachweisbar. Mit einem Kontrollantikörper ohne spezifische YB-1-Bindung ("Großbritannien", AB9, siehe Tabelle unten) sowie mit dem Anti-Maus Zweitantikörper alleine sind die Banden bei 16 und 23 kDa nicht nachweisbar.
**Fig. 11** zeigt den immunologischen Nachweis von YB-1 im Serum bei gesunden Probanden (Spuren 1 bis 3), zwei Patienten mit fulminanter Sepsis (Spuren 4 und 5) sowie von 8 Patienten mit metastasierenden Tumorerkrankungen verschiedener Organsysteme und Histologien (Spuren 6 bis 13). In Spur 14 ist eine Kontrollreaktion mit dem Erst-Antikörper allein aufgetragen, um unspezifische Banden nachzuweisen. In Spur 15 ist eine Kontrollreaktion mit dem Zweit-Antikörper allein aufgetragen, um durch den Zweit- Antikörper auftretende unspezifische Banden nachzuweisen. Durch Verwendung eines polyklonalen, gegen das Volllängen-YB-1 Protein gerichteten Antikörpers sind das Volllängen-YB-1 (52 kDA) sowie eine zweite Hauptbande eines YB-1 Fragments bei 28 kDa nachweisbar. Neben diesen, bei allen Proben sichtbaren YB-1 Fragmenten, ist ein kleines, ca. 14 kDa großes YB-1 Fragment bei sämtlichen Tumorpatienten nachweisbar, jedoch nur angedeutet in einem Patienten mit fulminanter Sepsis (Spur 4). Der Nachweis dieses Fragments in höherer Konzentration lässt somit auf das Vorliegen einer Tumorerkrankung schließen, wobei keine Zuordnung zu der Genese der Tumorerkrankung gemacht werden kann.
**Fig. 12** zeigt den Nachweis einer direkten Interaktion von GFP-YB-1 mit HA-getaggtem Notch in der Immunpräzipitation. Weitere Einzelheiten dazu siehe Ausführungsbeispiel 10.

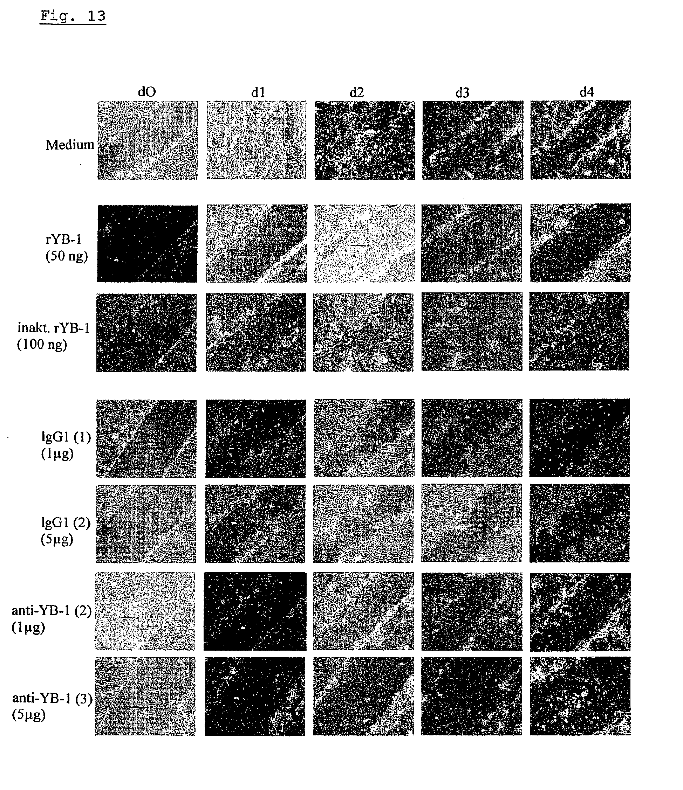

**Fig. 13** zeigt die Ergebnisse einer Inkubation von Keratinozyten mit rekombinanten YB-1 als auch mit anti-YB-1 Antikörpern nach einem Scratch-Versuch. Die Zeile 1 mit der Bezeichnung "Medium" zeigt dabei die Kontroll-Inkubation des Keratinozytenrasens mit Medium ohne YB-1 oder anti-YB-1 Antikörpern. Die Zeile 2 zeigt die Inkubation des Zellrasens mit rekombinanten YB-1 (rYB-1 (50 ng)), die Zeile 3 die Inkubation mit thermisch inaktiviertem rekombinanten YB-1 (rYB-1 (100 ng)), die Zeilen 4 und 5 die Inkubation mit den Kontroll-IgG Antikörpern (IgG1 (1) (1 µg) und IgG1 (2) (5 µg)) und die Zeilen 6 und 7 die Inkubation mit anti-YB-1 Antikörpern (anti-YB-1 (2) (1 µg) und anti-YB-1 (3) (5 µg)). Weiter Einzelheiten dazu siehe Ausführungsbeispiel 8.

### Beispiele

Im Folgenden werden die in den Beispielen verwendeten Antikörper tabellarisch zusammengefasst. Dabei wird insbesondere dargestellt, gegen welchen Teil des Volllängen YB-1-Proteins sich der Antikörper richtet oder gegen welches Fragment/e des YB-1-Proteins.

**Tabelle 1:**

| Antikörperbezeichnung | Antikörper (AB)-typ monoklonal od. polyklonal) | AB richtet sich gegen (welche Fragmente und/oder Fragmente ung/oder Volllängen YB-1) | Quelle |
|---|---|---|---|
| AB1 | polyklonal | C-Terminus des YB-1-Proteins | Mertens P.R., et al., J. Am. Soc. Nephrology, 1999, Vol.10, S.2480-2487 |
| AB2 (anti-Hasen HRP-konjugiertes F(ab')2-Fragment) | polyklonal | Zweit-Antikörper | Amersham Biosciences UK Limited, Buckinghamshire , UK |
| AB3 (Polyklonaler Hasen-Antikörper) | polyklonal | Volllängen YB-1-Protein | Mertens P.R. et al., The Journal of Biological Chemistry, 1997, Vol.272, Nr.36, S.22905-22912 |
| AB4 | monoklonal | Volllängen YB-1 | Hybridoma-Zelllinie "Deutschland" (beim DSMZ unter der Nummer DSM ACC 2703 hinterlegt) |
| AB5 (Anti-Maus Antikörper (PI-2000) | polyklonal | Zweit-Zweit-Antikörper | Amersham Biosciences UK Limited, Buckinghamshire , UK |
| AB6 (polyklonaler Hasenantikörper gegen HA-Tag) | polyklonal | HA-YB-1-Fusionsprotein (MWG 54 kDa) | Y-11 Santa Cruz Biotechnology Inc., Santa Cruz, California, USA |
| AB7 (polyklonaler Ziegen-Antikörper) | polyklonal | N-Terminus des Volllängen YB-1-Proteins | A-16X, Santa Cruz Biotechnology Inc., Santa Cruz, California, USA |
| AB8 | monoklonal | Volllängen-YB-1 | Hybridoma-Zelllinie "Italien" |
| AB9 | monoklonal | AB9 hat eine geringe Affinität für das Volllängen-YB-1 Protein und ein 26 kDa Fragment im Serum. Keine Detektion kleinerer YB-1 Fragmente wie 16 oder 23 kDa | Hybridoma-Zelllinie "Groß-britannien" |

### Beispiel 1

### Nachweis von sekretiertem YB-1 und seiner Fragmente nach Stimulation der monozytären Zelllinie MM-6 mit Lipopolysacchariden (LPS)

### Gewinnung von Mikrovesikeln aus MM-6 monozytären Zellen und Nachweis von YB-1

MM-6 monozytäre Zellen wachsen als Suspensionskultur in RPMI 1640 Medium, welches 10% (v/v) FCS (Fötales Kälberserum), 2 mM L-Glutamin, 100 µg/ml Streptomycin und 100 U/ml Penicillin-G enthält. Nach einem Waschvorgang in Phosphat gepufferter Salzlösung (PBS) werden die Zellen in Lipumin™haltigem (PAA Laboratories GmbH, Pasching, Österreich) RPMI Medium ohne FCS-Zugabe aufgenommen und gezählt. In Eppendorfgefäßen werden 2 x 10⁶ MM-6 Zellen/ml bei 37°C mit LPS und Adenosintriphosphat (1 mM) (beide Sigma Aldrich, St. Louis, USA) in den in den Fig. 1 und 2 angegebenen Konzentrationen für 4 Stunden inkubiert. Anschließend wird das Medium mit den Zellen für 10 min bei 330 x g zentrifugiert, damit die Zellen sedimentieren, und der Überstand wird dann erneut bei 2200 x g für 15 min zentrifugiert. Der Überstand nach der zweiten Zentrifugation wird mit eiskalter Trichloressigsäure zur Präzipitation der Proteine (einschließlich der Mikrovesikel) versetzt und über Nacht bei -20°C gelagert. Anschließend wird für 30 min bei 33.000 x g zentrifugiert und dann der Überstand verworfen. Das Sediment wird einmal mit eiskaltem nicht-vergälltem Ethanol (70%) gewaschen und in einer Vakuumzentrifuge getrocknet.

Anschließend wird das getrocknete Sediment in 25 µl Aqua dest. aufgenommen. Nach Zugabe von 25 µl denaturierendem Puffer wird es in einem SDS-Polyacrylamid-Gel (SDS-PA-Gel) (12,5%) elektrophoretisch aufgetrennt und YB-1 bzw. Fragmente von YB-1 mit den weiter unten unter dem Punkt "Antikörpernachweis" genannten Antikörpern (Mertens P.R., et al., J. Am. Soc. Nephrology, 1999, Vol.10, S.2480-2487) im Western-Blot (siehe unten) nachgewiesen. Neben dem Volllängen YB-1 (52 kDa) wurden dabei auch Fragmente von YB-1 mit Größen von 35, 32, 28 und 16 kDa nachgewiesen.

### Western-Blot Analyse

### Verwendete Lösungen:

Trenngel-Puffer (4-fach): 1,5 M Tris HCl, pH 8,8
Acryl/Bisacryl (30 %): 29,2 g Acrylamid, 0,8 g Bisacrylamid, 70 ml H₂O
Sammelgel-Puffer (4-fach) : 0,5 M Tris HCl pH 6,8
SDS (10 %): 10 g SDS in 90 ml H₂O
APS (10 %): 1 g Ammoniumperoxodisulfat in 9 ml H₂O
Elektrophorese-Puffer (10-fach): 30 g Tris HCl, 144 g Glyzin, 10 g SDS, auf 1000 ml H₂O auffüllen, alles auf pH 8,3 einstellen
Proben-Puffer (4-fach): 2,4 ml H₂O, 2,4 ml Sammelgel-Puffer, 2,0 ml Glyzerol, 2,0 ml SDS (10 %), 200 µl Bromphenolblau-Lösung(1 %), 40 µl EDTA-Lösung (0,5 M), 1 ml β-Mercaptoethanol
Transfer-Puffer: 3,03 g Trizma Base, 14,4 g Glyzin, 200 ml Methanol, mit H₂O auf 2000 ml auffüllen
TTBS-Lösung: 1916 ml H₂O, 60 ml NaCl-Lösung (5 M), 20 ml Tris-Lösung (1 M, pH 8), 4 ml Tween-20-Lösung (25 %)

Die Proteine werden elektrophoretisch aufgetrennt, auf eine Nitrozellulosemembran transferiert und mit anti-YB-1 Antikörpern (siehe unten stehende Liste unter dem Punkt "Antikörpernachweis") wie folgt detektiert:
Zuerst wurde ein 12,5 % SDS-PA-Gel in das *BioRad Minigel System* gegossen. Für 4 Trenngele (entsprechend etwa 25 ml) wurden nacheinander folgende Lösungen miteinander gemischt: H₂O 8 ml, Trenngel-Puffer (4-fach) 6,25 ml, Acryl/Bisacryl (30 %) 10,4 ml, SDS (10 %) 250 µl, TEMED 12,5 µl, APS (10 %) 125 µl.

Anschließend wurde das noch flüssige Trenngel sofort zwischen die Glasscheiben des *Minigel* Systems pipettiert, bis der Flüssigkeitsspiegel 1 cm unter dem oberen Glasplattenrand stand. Der restliche Raum wurde mit Wasser aufgefüllt und bei Raumtemperatur für 45 Minuten polymerisiert.

Für 4 Sammelgele wurden nacheinander folgende Lösungen miteinander vermischt: H₂O 6,1 ml, Sammelgel-Puffer (4-fach) 2,5 ml, Acryl/Bisacryl (30 %), 1,3 ml, SDS (10 %) 100 µl, TEMED 10 µl, APS (10 %) 50 µl.

Das überstehende Wasser zwischen den Glasplatten wurde dekantiert und der Glasspalt mit dem flüssigen Sammelgel aufgefüllt. Der Kamm wurde sofort eingeschoben und das Sammelgel für 45 Minuten bei Raumtemperatur polymerisiert.

In der Zwischenzeit werden die Proben aus den Zellextrakten bzw. nach Zentrifugation des Überstandes vorbereitet. 10 µl Probe wurden mit 10 µl denaturierendem Proben-Puffer (*Laemmle*) gemischt und für 3-5 Minuten bei 95 °C in einem Wasserbad inkubiert. Die Kämme werden aus dem polymerisierten Gel entfernt und die Kammer mit Elektrophorese-Puffer (1-fach konzentriert) aufgefüllt. Anschließend wird jede Probe kurz zentrifugiert, die Geltaschen mit einer Pipette vorgespült, jede Probe in eine Geltasche aufgetragen und für ca. 1 Stunde bei 150 Volt elektrophoretisch aufgetrennt.

Während der Elektrophorese werden die Vorbereitungen für den Transfer getroffen. Dazu wurde der Transfer-Puffer hergestellt und bei 4 °C vorgekühlt. Pro Gel wurden eine Nitrozellulosemembran und die doppelte Menge an *Whatman-*Papier zurechtgeschnitten. Die Gele wurden nach der Elektrophorese aus der Kammer genommen, Nitrozellulosemembran und *Whatman*-Papier in Transfer-Puffer angefeuchtet und in folgender Reihenfolge blasenfrei aufgeschichtet: *Whatman-*Papier, Nitrozellulosemembran, Gel, *Whatman*-Papier. Für den Transfer wird die Nitrozellulosemembran in der Transferkammer zur Anode gerichtet, die Kammer mit Transferpuffer aufgefüllt, ein Eisblock zur Kühlung einlegt und 1 Stunde bei 100 Volt geblottet.

### Antikörpernachweis

Nach dem Transfer werden die Membranen für 3 x 10 Minuten in TTBS-Lösung gewaschen, in 2 % BSA-Lösung (gelöst in TTBS) für 2 Stunden bei Raumtemperatur geblockt und erneut 3 x 10 Minuten in TTBS gewaschen. Die Membranen werden anschließend mit dem Erst-Antikörper in einer Verdünnung von 1:2000 über Nacht bei 4 °C inkubiert. Hierbei werden unterschiedliche Anti-YB-1 Antikörper eingesetzt (siehe untenstehende Auflistung). Nach einem weiteren Waschvorgang werden die Membranen mit dem Zweit-Antikörper inkubiert (siehe untenstehende Auflistung).

Folgende Antikörperkombinationen ([Erst-Antikörper] : [Zweit-Antikörper]) werden eingesetzt, um YB-1 und Fragmente von YB-1 nachzuweisen:
- Polyklonaler Hasenantikörper gegen den YB-1 C-Terminus (AB1) (Mertens P.R., et al., J. Am. Soc.-Nephrology, 1999, Vol.10, S.2480-2487): anti-Hasen HRP-konjugiertes F(ab')2-Fragment (AB2) (*Amersham Biosciences UK Limited, Buckinghamshire, UK*) aus dem Esel, in einer Verdünnung von 1:5.000 für 2 Stunden bei RT inkubiert.
- Polyklonaler Hasen-Antikörper gegen Volllängen-YB-1 Protein (AB3) (Mertens P.R. et al., The Journal of Biological Chemistry, 1997, Vol.272, Nr.36, S.22905-22912): anti-Hasen HRP-konjugiertes F(ab')2-Fragment (AB2) (*Amersham Biosciences UK Limited, Buckinghamshire, UK*) aus dem Esel, in einer Verdünnung von 1:5000 für 2 Stunden bei RT inkubiert.
- Monoklonaler Anti-YB-1 Antikörper (AB4 und AB8) : Anti-Maus Antikörper (AB5) (PI-2000, Amersham *Biosciences UK Limited, Buckinghamshire, UK*) aus dem Esel, in einer Verdünnung von 1:20.000 für 2 Stunden bei RT inkubiert. Die monoklonalen Antikörper "Deutschland" (AB4) und "Italien" (AB5) wurden gegen das rekombinant in Bakterien hergestellte Volllängen-YB-1 Protein generiert, indem Mäuse immunisiert wurden und nach 2 Booster-Injektionen mit dem rekombinanten Protein Milzlymphozyten nach dem folgenden Protokoll isoliert und mit Hybridoma-Zellen fusioniert wurden.

### Herstellung monoklonaler Antikörper

*Immunisierung:* BALB/c Mäuse wurden im Alter von 8 Wochen mit gereinigtem Antigen (rYB-1 Proteine, siehe Mertens et al., The Journal of Biological Chemistry, 1997, Vol.272, Nr.36, S.22905-22912) immunisiert. Die Dosis beträgt 150 µg/Maus. Für die erste Immunisierung werden die Antigene in komplettem Freund schen Adjuvans/CFA (Sigma) emulgiert (Wasser/Öl-Emulsion, Hurn und Chantler, 1980) und den Mäusen in einem Volumen von 300 µl subkutan injiziert. Vor der Immunisierung wird den Mäusen Blut aus der Schwanzvene entnommen (Prä-Immunserum). 4 Wochen nach der ersten Immunisierung wird den Mäusen Blut entnommen (Immunserum). Die Antikörpertiter in diesen Seren werden in einem semiquantitativen Enzym-Immuno-Assay (Slot-Blot) bestimmt. Mäuse mit einem hohen Antikörpertiter werden 6-8 Wochen nach der ersten Immunisierung und 2 Wochen später erneut immunisiert (Boosterimmunisierung). Hierbei werden den Tieren 100 µg des rYB-1 in PBS intravenös injiziert. 3-5 Tage nach der letzten Immunisierung wird die Fusion durchgeführt.

### Präparation der Milzzellsuspensionen zur Gewinnung von

*Milzzellen:* Den Mäusen wird nach zervikaler Dislokation die Milz unter sterilen Bedingungen entnommen und in einer Petrischale in HBSS (*Hanks buffered salt solution*) vorsichtig mit 2 Pinzetten zerzupft. Zellklumpen und Bindegewebsreste werden durch Filtration über ein feines Drahtsieb entfernt. Anschließend werden die Zellen dreimal in HBSS gewaschen (Zentrifugation für 10 min mit 200 g bei Raumtemperatur). Für die Klonierung der Hybridome wird rekombinantes IL-6, (100 U/ml, Roche) eingesetzt.

### Zellfusion: Für die Zellfusion wird ein modifiziertes

Protokoll der ursprünglich von Köhler und Milstein (Köhler und Milstein, Biotechnology, 1992; Vol.24, S.524-526) beschriebenen Methode verwendet: 10⁸ gewaschene Milzzellen aus einer immunisierten Maus und 2 bis 5×10⁷ 8-Azaguaninresistente Myelomzellen (X63-Ag8/653) werden in einem 50 ml Zentrifugationsröhrchen gemischt. Die Myelomzellen werden in großen Zellkulturflaschen (225 cm²) 10 Tage zuvor herangezogen. Anschließend wird ihre Zellzahl in einer Neubauer-Zählkammer bestimmt. Das Röhrchen wird mit GKN-Medium (8 g/l NaCl, 0,4 g/l KCl, 1,77 g/l Na₂HPO₄, 0,69 g/l NaH₂PO₄, 2 g/l Glukose) aufgefüllt und 10 min mit 200 xg bei Raumtemperatur zentrifugiert. Der Überstand wird abgesaugt. 0,5 ml 50% PEG-Lösung (Sigma) werden über einen Zeitraum von 1 min tropfenweise zugegeben. Das Sediment wird hierbei durch vorsichtiges Klopfen resuspendiert. Um das PEG zu verdünnen werden nach 90 Sekunden 5 ml GKN-Medium über einen Zeitraum von 5 min zugegeben. Nach einer Ruhephase von 10 min werden große Klumpen durch vorsichtiges Pipettieren mit einer 10 ml-Pipette aufgelöst. Die Zellsuspension wird anschließend in HAT-Medium (Medium mit Hypoxanthin, Aminopterin und Thymidin zur Kultivierung von Zellhybriden,DMEM (Gibco)), 2 mM L-Glutamin, 5×10⁻⁵ M ß-Mercaptoethanol, 2% (v/v) 50×HAT, 1% (v/v) nicht essentielle Aminosäuren, 10% (v/v) fetales Kälberserum, 100 µg/ml Streptomycin und 100 U/ml Penicillin) verdünnt und auf 6 Mikrotiterplatten (96 Kammer) verteilt. Die Zelldichte beträgt ca. 1×10⁵ pro Kammer in einem Volumen von 100 µl. Die Hybridome werden in wöchentlichem Abstand mit HAT-Medium versorgt. Nach etwa 2 Wochen ist die Mehrzahl der Hybridome expandiert und besitzt eine Zellzahl von ca. 5×10³ -10⁴. Zu diesem Zeitpunkt werden die Überstände mit einem Dot-Blot-Enzym-Immunoassay auf den Gehalt an spezifischen Antikörpern getestet. Hierfür werden jeweils 100 µl/Kammer der Überstände von der Mikrotiterplatten entnommen und eingesetzt. Positiv getestete Hybridome werden expandiert.

*Klonierung nach dem Prinzip der limitierten Verdünnung:* Um die Monoklonalität eines antikörperproduzierenden Klons zu gewährleisten, bietet sich die von Coller und Coller (Hybridoma, 1983; Vol.2, S.91-96.) beschriebene Methode der Limited-Dilution an. Mittels Neubauer Zellkammer wird die Zellzahl der Klone bestimmt und die Zellkultur mit Medium verdünnt, so dass sich statistisch auf jede zweite Kammer einer 96 Kammer-Platte nur eine Zelle verteilt. Die Kulturen wird erneut expandiert und die Überstände aus diesen Kulturen werden nach ca. 2 Wochen erneut im Enzym-Immuno-Assay (Western Blot, ELISA) auf die Produktion spezifischer Antikörper getestet. Positive Klone werden expandiert, näher charakterisiert und zur Sicherung in flüssigem Stickstoff eingefroren. Zum Einfrieren werden die Hybridomzellen geernet, abzentrifugiert, mit einer Zelldichte von 1× 10⁶ Zellen/ml in eiskaltem Einfriermedium (DMEM mit 10% (v/v) DMSO, 20-30% (v/v) fötales Kälberserum) resuspendiert und in Stickstoff gelagert.

Eine Isotypisierung des "Deutschland"-Antikörpers (AB4) ergab eine Zugehörigkeit zur IgG2b Subklasse. Die Antikörer mit der Bezeichnung "Italien" (AB8) und "Großbritannien" (AB9) sind auf gleiche Art und Weise hergestellt worden.

Die Detektion erfolgt mittels der "Enhanced *Chemiluminiscence*"-Reaktion (ECL™, Amersham *Biosciences UK Limited, Buckinghamshire, UK*) und anschließender Röntgenfilm-Exposition.

### Ergebnisse:

In Fig. 1A wird die Induktion der Sekretion von Volllängen YB-1 (52 kDa) bei Zugabe von 1 ng/ml LPS deutlich (Spur 4).

Niedrigere Konzentrationen (10⁻² bis 10⁻¹ ng/ml) und höhere Konzentrationen (10 bis 10⁴ ng/ml) führen nicht bzw. nur zu einer geringen Sekretion, wie zum Beispiel bei einer LPS-Konzentration von 10⁴ ng/ml zu sehen ist (Spur 8).

Eine weitere Testreihe in einem weiter eingegrenzten Konzentrationsbereich von 0,1 bis 10 ng/ml (Fig. 1B) zeigt, dass Volllängen YB-1 von den MM6-Zellen bei LPS-Konzentrationen von 1,0 bis 7,5 ng/ml sezerniert wird. Bei längerer Inkubation der Membran aus dem Western-Blot mit den Antikörpern ist eine konstitutive Expression von Volllängen YB-1 auch bei niedrigen LPS-Konzentrationen (0,25 bis 0,75 ng/ml) zu erkennen (Fig. 2). Zudem wird eine Sekretion von YB-1 Spaltprodukten bei LPS-Konzentrationen zwischen 1,0 und 7,5 ng/ml deutlich. Die Spaltprodukte von YB-1 weisen Größen von 35, 32, 28 und 16 kDa auf.

In Fig.1 zeigt sich außerdem, dass bei LPS-Konzentrationen von 1 ng/ml (Fig.1A) bzw. 1 ng/ml bis 7,5 ng/ml (Fig.1B) der *macrophage migratory inhibitory factor* (MIF) und Aktin vorhanden sind. Dies belegt die Spezifität des Sekretionsmechanismus und dass das nachgewiesene YB-1 nicht etwa aus lysierten Zellen stammt.

### Beispiel 2

### Vergleich des Molekulargewichtes (MGW) von intrazellulär exprimierten und sekretiertem YB-1

In diesem Versuch werden die Unterschiede im Molekulargewicht zwischen rekombinant in *E*. *coli* hergestelltem YB-1 (rYB-1), nukleärem YB-1 (NE) und nach LPS-Stimulation von MM-6-Zellen sekretiertem YB-1 (LPS/sup.) untersucht.

Die Beschreibung des Versuchsaufbaus für die LPS-Stimulation von MM-6-Zellen und die Bestimmung des sekretierten YB-1 finden sich in Beispiel 1. Die Herstellung von rekombinanten YB-1 und die Isolation von nukleärem YB-1 erfolgt nach der Methode, wie sie in den Artikeln von Mertens et al., J. Biol. Chem., 1997, Vol.272, S.22905-22912; Mertens P.R. et al., J.Biol.Chem., 1998, Vol.273, S.32957-32965 beschrieben worden ist. Der anschließend mit den Proben von nukleärem YB-1, rekombinantem YB-1 und nach LPS-Stimulation sekretiertem YB-1 durchgeführte Western-Blot wird gemäß der Beschreibung zu Beispiel 1 durchgeführt. Die im Western-Blot verwendeten Antikörper richten sich zum einen gegen
- den C-Terminus (anti-C (AB1); s.o. Mertens P.R., et al., J. Am. Soc. Nephrology, 1999, Vol.10, S.2480-2487)
- den N-Terminus (anti-N (AB7), Anti-YB-1 Antikörper von Santa Cruz, der in einer Verdünnung von 1:2000 eingesetzt wird) und
- das Volllängen YB-1 Protein (anti-w (AB3); s.o. Mertens P.R. et al., The Journal of Biological Chemistry, 1997, Vol.272, Nr.36, S.22905-22912) (weitere Details siehe Beispiel 1). Die Gewinnung von nuklearen Zellextrakten wird im Folgenden näher beschrieben.

### Gewinnung von nukleären Zellextrakten (NE):

### Verwendete Lösungen:

Hypotoner Puffer: 10 mM HEPES pH 7,9, 1,5 mM MgCl₂, 10 mM KCl, 0,2 mM PMSF, 0,5 mM DTT, 1 mM Natriumorthovanadat.
Puffer mit niedriger Salzkonz.: 20 mM HEPES pH 7,9, 25 Vol.-% Glyzerol, 1,5 mM MgCl₂, 20 mM KCl, 0,2 mM EDTA, 0,2 mM PMSF, 0,5 mM DTT, 1 mM Natriumorthovanadat.
Puffer mit hoher Salzkonz.: 20 mM HEPES pH 7,9, 25 Vol.-% Glyzerol, 1,5 mM MgCl₂, 1,0 M KCl, 0,2 mM EDTA, 0,2 mM PMSF, 0,5 mM DTT, 1 mM Natriumorthovanadat.
Dialyse-Puffer: 20 mM HEPES, 20 Vol.-% Glyzerol, 0,1 M NaCl, 0,2 mM EDTA, 0,2 mM PMSF, 0,5 mM DTT.

Die Gewinnung von nukleären Extrakten lehnt sich an die von Dignam et al. beschriebenen Methode an (Methods Enzymol., 1983; Vol.101, S.582-598). Die Zellen werden in 250 ml-Zellkulturflaschen ausgesät und bis zu einer Konfluenz von 80 % in komplettem RPMI-Medium mit 10% FCS kultiviert. Das Medium wird dekantiert, die Zellen 1x mit eiskaltem PBS gewaschen und anschließend in 10 ml eiskaltem PBS mit einem Zellschaber vorsichtig abgelöst. Die folgenden Arbeitsschritte werden stets auf Eis bzw. bei 4 °C durchgeführt: Zentrifugation der Zellsuspension für 10 Minuten bei 3.000 Umdrehungen pro Minute (rpm), Dekantierung des Überstands und Aufnehmen des ZellSediments im 5-fachen Sedimentvolumen hypotonen Puffer (siehe oben). Die Suspension wird erneut für 5 Minuten bei 3.000 rpm zentrifugiert. Das Sediment wird wieder in hypotonem Puffer aufgenommen (3-faches Sedimentvolumen), 10 Minuten auf Eis inkubiert und bei 4.000 rpm für 15 Minuten zentrifugiert. Der Überstand wird nach der Zentrifugation verworfen, das Sediment in Puffer mit niedriger Salzkonzentration aufgenommen (1/2 Sedimentvolumen) und vorsichtig Puffer mit hoher Salzkonzentration (1/3 des Gesamtvolumens) hinzupipettiert. Anschließend werden die Extrakte jeweils für 30 Minuten geschüttelt und bei 14.000 rpm zentrifugiert. In der Zwischenzeit werden Dialyseschläuche in EDTA 0,05 M aufgekocht und gewässert. Nach der Zentrifugation wird der Überstand in die Dialyseschläuche pipettiert und in 2 Liter vorgekühltem Dialyse-Puffer über Nacht bei 4 °C unter ständigem Rühren dialysiert. Am nächsten Morgen wird der Dialyse-Puffer gewechselt und für weitere 2 Stunden dialysiert. Anschließend werden die Proben aliquotiert, mit flüssigem Stickstoff schockgefroren und bei -80 °C gelagert.

### Ergebnisse:

Die erste Reihe im Gel des in Fig. 3 dargestellten Western-Blots dient der Negativkontrolle (d.h. es wurde kein primärer Antikörper verwendet). Es zeigt sich eine geringere Mobilität von sekretiertem YB-1 (Spuren 1, 5 und 8) im Vergleich zu YB-1 nukleären Ursprungs (Spuren 3, 6 und 9). Das prokaryontisch hergestellte YB-1 (rYB-1) unterscheidet sich nicht nur in seinem Molekulargewicht von 46 kDa vom eukaryontisch hergestellten YB-1 (LPS/sup.; 52 kDa), es ist zudem als höhermolekulare Bande (88 kDa) nachweisbar (Spur 4, 7 und 10), am ehesten aufgrund einer Homomultimerisierung. Zudem können Fragmente mit einer Größe von 38, 36, 35, 33 und 22 kDa nachgewiesen werden. Mit dem gegen Volllängen-YB-1 Protein hergestellten Antikörper ist bei rekombinantem YB-1 zudem ein 25 kDa Fragment nachweisbar (Spur 7).

Zum Unterschied zwischen sekretiertem und intrazellulärem YB-1 ist den Erfindern derzeit nur bekannt, dass sekretiertes YB-1 nicht in glykosylierter Form vorliegt, wie es für Sekretionsprodukte des klassischen Weges beschrieben ist. Es existieren Daten, welche den Sekretionsweg von YB-1 näher beleuchten (nicht dargestellt). Nach bisherigem Wissen erfolgt die Sekretion aktiv über ABC-Transporter, was nach aller Wahrscheinlichkeit dem alternativen Sekretionsweg zuzuordnen ist. Zudem fällt bei der Analyse der Fig. 3 auf, dass das sekretierte YB-1 ungefähr um 5 kDa größer ist als das nukleäre YB-1 Protein (ca. 57 kDa) und nicht nur als Volllängenrotein, sondern auch in Fragmenten vorliegt. Wie in Fig. 3 zu erkennen, liegt intrazelluläres YB-1 als Doppelbande bei 50 und 52 kDa vor (siehe z.B. Spur 9).

### Beispiel 3

### Nachweis von in Mikrovesikeln sekretiertem YB-1 und

### Fragmenten von YB-1 nach Stimulation einer konditionell HA-YB-1 exprimierende Mesangialzelllinie mit dem

### Blutplättchenwachstumsfaktor PDGF-B (engl. "platelet-derived growth factor")

Das folgende Beispiel zeigt, dass Zellen einer konditionell HA-YB-1 exprimierenden Mesangialzelllinie bei Stimulation mit PDGF-B YB-1 und Fragmente von YB-1 sekretieren. Der Versuch belegt außerdem, dass es sich bei dem sekretiertem YB-1-Protein nicht um ein anderes Mitglied der Kälteschockproteinfamilie handelt, zu der YB-1 gehört.

Dafür werden in diesem Beispiel primäre Mesangialzellen von Ratten (Mertens et al., Hypertension, 1998, Vol.32, S.945-952) verwendet. Sie wachsen in RPMI 1640 Medium, dem 10% fötales Kälberserum, 2 mM L-Glutamin, 1 mM Na-Pyruvat, nicht essentielle Aminosäuren, 5 mg/l Insulin, 3.4 µg/l Natriumselenit, 2.8 mg/l Transferrin, 100 µg/ml Streptomycin und 100 U/ml Penicillin zugesetzt worden ist, bei 37°C unter einer H₂O-gesättigten Atmosphäre, die 5% CO₂ enthält.

Für die Induktion der Zellen zur Sekretion von YB-1 lässt man die Zellen in 75 cm² Kulturschalen bis zu einer 80%-igen Konfluenz in Gegenwart von Hygromycin (200 µg/ml) und Geneticin (400 µg/ml) heranwachsen. Vor der Stimulation mit PDGF-B werden die Zellen für 24 h mit MCDB-Medium (Sigma, St. Louis, MO, USA) behandelt, damit diese das Wachstum einstellen. PDGF-B wird bis zu einer Endkonzentration von 50 ng/ml dem RMPI-Medium zugegeben. Nach der Kultivierung werden die Zellen mit PBS ohne Calcium und Magnesium/EDTA (1 mM) von dem Zellkulturflaschenboden abgelöst und in ein steriles 50 ml Falcon-Röhrchen überführt, anschließend für 10 min bei 1000 U zentrifugiert und der Überstand abgenommen. Das ZellSediment wird in 5 ml PBS resuspendiert, die Zellzahl in der Neubauer-Kammer ausgezählt und die Zellen erneut bei 1000 U/min für 10 min zentrifugiert. Das ZellSediment wird in RPMI-Medium ohne Serumzusatz aufgenommen, so dass die Zellzahl 5x10⁵ Zellen/100 µl Medium beträgt. 200 µl werden in ein Eppendorfgefäß pipettiert und im Thermoblockschüttler bei 37°C mit PDGF-B (20 ng/ml PDGF-B) inkubiert.

Nach 4 Stunden Inkubationszeit werden die Eppendorfgefäße für 15 min bei 330 x g bei 4°C zentrifugiert, um die Zellen zu sedimentieren. 50 µl vom Überstand (S1) werden zur LDH Messung asserviert. Der verbleibende Überstand wird durch einen 0.2 µm Filter (Qualilab, Merck, Bruchsal) gegeben und bei 100.000 x g für 210 min zentrifugiert. Der Überstand (S2) sowie das Sediment (P2) werden getrennt in denaturierendem Laemmli-Puffer aufgenommen und sämtliche Proben in einem 10% SDS-PA Gel aufgetrennt (s. Fig. 5). Die sezernierten Mikrovesikel sind in Sediment P2 enthalten. Eine schematische Darstellung der vorgenannten Schritte, die zur Gewinnung von Mikrovesikeln im Medium führen, ist in Fig. 4 dargestellt. Der immunologische Nachweis des YB-1-Proteins im anschließenden Western-Blot (Einzelheiten zum Western-Blot siehe Beispiel 1) erfolgt mittels eines Anti-HA-Antikörpers (AB6).

### Ausführungsbeispiel 4

### Nachweis von YB-1 und seiner Spaltprodukte im Urin von Patienten mit progredienter IgA-Nephritis

Die Ausführungsbeispiele 4 bis 5 und 7 bis 8 verdeutlichen die Korrelation der Sekretion des YB-1 Proteins und Fragmenten des YB-1 Proteins durch die Zellen mit dem Krankheitsbild der untersuchten Patienten. Diese Beispiele zeigen die Möglichkeit, das Sekretionsmuster für YB-1 und dessen Fragmente durch die Zelle für die eindeutige Zuordnung zu einem entzündlichen Vorgang bei einem Patienten zu nutzen. Dadurch können die Entstehung und der Verlauf von entzündlichen Erkrankungen im Patienten besser bestimmt werden.

In diesem Beispiel wird das Sekretionsmuster von YB-1 und Fragmenten von YB-1 bei einer fortschreitenden (progredienten) IgA-Nephritis bei 2 Patienten dargestellt (Fig. 6). Der Nachweis von YB-1 und seiner proteolytischen Fragmente im Western-Blot (näheres zum Western-Blot in Beispiel 1) erfolgt mit einem gegen das gesamte YB-1 Protein hergestellten polyklonalen Antikörper (AB3) (Mertens P.R. et al., The Journal of Biological Chemistry, 1997, Vol.272, Nr.36, S.22905-22912).

Bei zwei ausgewählten Patienten mit einer Proteinurie, die eine Begleiterscheinung fast aller Nierenerkrankungen ist, von über 1,5 g/d (in Fig. 6 mit A und B gekennzeichnet) zeigt sich bei diesen immunologischen Untersuchungen ein charakteristisches Sekretionsmuster für YB-1. Das mit dem polyklonalen Antikörper markierte YB-1 Protein und seine proteolytischen Fragmente treten bei beiden Patienten in einem gleichartigen charakteristischen Bandenmuster im Gel auf, wobei Proteinbanden bei Molekulargewichten von 52, 28, 23, 16 und 8 kDa beobachtet werden können (s. Fig. 6, Spuren 1 und'4). Die Bande bei 52 kDa stellt dabei das Volllängenprotein YB-1 dar. Durch die Multimerisierung von YB-1 mit sich selbst und anderen kleineren Fragmenten von YB-1 treten auch Banden mit Molekulargewichten über 52 kDA auf. Bei gleichbleibendem Krankheitsverlauf bei den Patienten sind diese Befunde sequenziell in etwa 3 monatigen Abständen durch die Untersuchung von weiteren Spontan-Urinproben bestätigt worden (Fig. 6, Spuren 2 und 3 für Patient **A** bzw. 5 und 6 für Patient **B**)**.** In allen Fällen trat immer das nahezu gleiche Bandenmuster im Gel auf, was auf die gleichbleibende Sekretion von YB-1 und dessen proteolytischen Fragmente hinweist.

In Fig. 7 zeigt Gel von 12 Urinproben, die von Patienten mit IgA-Nephritis gewonnen wurden,. Die Konzentrationsangaben für Serumkreatinin unter den einzelnen Spuren des Gels geben die aktuelle Nierenfunktion an, die delta-Kreatinin-Werte im Serum zeigen an, wie sich die Nierenfunktion entwickelt. Der Verlauf der Konzentrationsänderung der Kreatininkonzentration ist über 2 Jahre bei den Patienten beobachtet und die Anordnung der Proben auf dem Gel für die Patienten nach steigender Kreatininkonzentration geordnet worden. Bei dem immunologischen Nachweis mit einem gegen das Volllängen-YB-1 Protein gerichteten Antikörper (AB3) (Mertens P.R. et al., 1997, The Journal of Biological Chemistry, Vol.272, Nr.36, S.22905-22912) fällt auf, dass neben dem Volllängen-YB-1 Protein bei 52 kDa auch Fragmente mit den Molekulargewichten von 28, 23, 16 und 8 kDa nachweisbar sind. Nach Probenasservierung sind die Patienten bezüglich ihres klinischen Verlaufs evaluiert und der Anstieg des Serumkreatinins angegeben worden. Während die Nierenfunktion bei den meisten Patienten stabil ist, ist es bei den beiden Patienten mit Nachweis der 23, 16 und 8 kDa YB-1 Fragmenten zu einer Progression hin zum terminalen Nierenversagen und Dialysepflichtigkeit gekommen. Hierbei fällt zudem auf, dass der Nachweis von YB-1 unabhängig vom Ausmaß der Proteinurie erfolgt, wie anhand der Vergleichsdaten zur Proteinurie unter der Abbildung des Gels zu erkennen ist. Zur Kontrolle wird in Spur 13 die Urinprobe eines der beiden Patienten mit progredienter Niereninsuffizienz (Spur 10) ausschließlich mit dem Zweit-Antikörper inkubiert. Die niedermolekularen Banden sind in diesem Fall nicht zu erkennen.

In Fig. 8 ist exemplarisch der Verlauf bei einem Patienten mit bioptisch gesicherter IgA-Nephritis, bei dem sich das YB-1 Muster im Urin über Wochen änderte, dargestellt. Hierbei fällt auf, dass die niedermolekularen Banden verschwinden und es zu einer Wiederherstellung der Nierenfunktion kommt. Der Patient wurde zum Zeitpunkt des akuten Nierenversagens (Zeitpunkt 0) immunsuppressiv behandelt.

### Ausführungsbeispiel 5

### Nachweis des YB-1 Proteins und seiner Proteinfragmente als Indikator für eine akute Nierentransplantationsabstoßung

Der monoklonale Antikörper "Deutschland" (AB4) ist zum Nachweis von YB-1 im Western-Blot verwendet worden. Dieser monoklonale Antikörper erkennt die kleineren Fragmente bei 8, 16 und 23 kDa nicht. Die Urinproben werden sequenziell nach erfolgter Nierentransplantation bei den untersuchten 6 Patienten (A-F) gesammelt. Bei den in Fig. 9 mit AR bezeichneten Patienten A und B liegt eine durch Biopsie gesicherte Abstoßurigsreaktion (AR) vor. Bei den Patienten C und D liegen weder eine Abstoßungsreaktion noch irgendwelche weiteren Komplikationen vor (No AR). Bei den Patienten E und F sind zwar keine Abstoßungsreaktionen aufgetreten, jedoch gibt es andere Komplikationen (comp., Ø AR) wie Peritonitis und Katheter-assoziierte Sepsis. Parallel zur Bestimmung der Anwesenheit von YB-1 und dessen Fragmente werden auch Zytokine und andere Markerproteine für Abstoßungsreaktionen bestimmt.

Die Ergebnisse in Fig. 9 zeigen, dass die Reaktion des Körpers nach der Transplantation sich eindeutig im Sekretionsprofil von YB-1 und dessen Proteinfragmente widerspiegelt. Wie beim Patienten A in Fig. 9 zu erkennen ist, sind die kleineren Fragmente (MGW von etwa 27 kDa) schon nachweisbar, bevor das für die Abstoßungsreaktion als Frühmarker evaluierte Granzym B signifikant angestiegen ist. Dieses Ergebnis zeigt außerdem, dass der Nachweis von YB-1 und dessen Fragmente unabhängig von dem Ausmaß der Proteinurie ist.

Die vorhergehenden Beispiele zeigen, dass sich dem behandelnden Arzt durch die Analyse des YB-1 Sekretionsmusters eine schnelle Möglichkeit bietet, die entzündliche Vorgänge und die Ursache dieser entzündlichen Vorgänge zu bestimmen.

### Ausführungsbeispiel 6

### Sekretion von YB-1 durch monozytäre Zellen und Makrophagen nach Stimulation mit LPS

Wie in Figur 10A erkenntlich ist, beginnen auch primäre humane Monozyten und Makrophagen, die nach einem Standardprotokoll mittels *buffy coats* aus dem Blut von gesunden Spendern gewonnen worden sind, bei einer Inkubation mit LPS YB-1 zu sekretieren. In dem Blot wurde ein polyklonaler Antikörper (AB3), der gegen das gesamte YB-1 Protein hergestellt wurde, zur Detektion eingesetzt.

### Ausführungsbeispiel 7

### Nachweis von YB-1 und Fragmenten von YB-1 in Verbindung mit malignen Erkrankungen

Fig. 11 zeigt den immunologischen Nachweis von YB-1 im Serum bei gesunden Probanden (Spuren 1 bis 3), zwei Patienten mit fulminanter Sepsis (Spuren 4 und 5) sowie von 8 Patienten mit metastasierenden Tumorerkrankungen verschiedener Organsysteme und Histologien (Spuren 6 bis 13). Die Diagnosen umfassen Pankreas-Karzinom (Pa.-CA), nicht-kleinzelliges Bronchialkarzinom (NSC-BC), Rektum-Karzinom (Re.-CA), Mamma-Karzinom (Ma.-CA), Larynx-Karzinom (La.-CA) und Adeno-Karzinom ohne Nachweis eines Primärtumors (Ad.-CA). In den Spuren 14 und 15 sind die Proben eines Tumorpatienten mit Pankreas-Karzinom erneut aufgetragen. Bei Spur 14 erfolgte eine Inkubation mit Erst-Antikörper gegen YB-1, in Spur 15 eine Inkubation ausschließlich mit dem Zweitantikörper, um unspezifische Banden, die durch den Zweitantikörper bedingt sind, nachzuweisen. Es zeigen sich hierbei zwei schwache unspezifische Banden bei 50 und 26 kDa. Durch Verwendung eines polyklonalen, gegen das Volllängen-YB-1 Protein hergestellten Antikörpers (AB3) sind das Volllängen-YB-1 (52 kDA) sowie eine zweite Hauptbande eines YB-1 Fragments bei 28 kDa nachweisbar. Neben diesen, bei allen Proben sichtbaren YB-1 Fragmenten, ist ein kleines, ca. 14 kDa großes YB-1 Fragment bei sämtlichen Tumorpatienten nachweisbar, jedoch nur angedeutet in einem Patienten mit fulminanter Sepsis (Spur 4). Der Nachweis dieses Fragments in höherer Konzentration lässt somit auf das Vorliegen einer Tumorerkrankung schließen.

### Ausführungsbeispiel 8 (nicht erfindungsgemäß)

### Einfluss von YB-1 und Antikörper gegen YB-1 auf die Mobilität von Keratinozyten

Humane Keratinozyten werden von Hautbiopsaten adulter Spender gewonnen, indem die Hautproben drei Mal mit PBS-Lösung, der Penizillin und Amphotericin B zugegeben worden ist, gewaschen werden. Die Proben werden mit 70% Ethanol gewaschen und in Stücke von 1 cm² geschnitten. Die Epidermis wird durch Inkubation mit caseinolytischer Dispase (50 U/ml, Collaborative, Bedford, MA, USA) für 2h bei 37°C aufgetrennt. Nach Inkubation in 0,25 mg/ml Trypsin/EDTA (Cambrex, Walkersville, MD, USA) für 30 Minuten bei 37°C werden die Keratinozyten als Einzelzellsuspension durch vorsichtiges Pipettieren freigesetzt. Nach Neutralisieren des Trypsins mit Trypsin Neutralisationslösung (TNS, Cambrex, Walkersville, MD, USA) werden die Zellen in KGM Medium (Cambrex, Walkersville, MD, USA) aufgenommen und in Zellkulturflaschen ausgesät und kultiviert.

Nach Konfluenz der Zellen wird ein "Scratch" durchgeführt, d.h. ein Defekt in den Zellrasen gesetzt (Draper BK et al., J Cell Biochem., 2003, Vol.89, S.1126-37). Nach dem Spülen der Zellen mit KGM Medium wird rekombinantes YB-1 bzw. die Antikörper in den angegebenen Konzentrationen zugegeben.

### Ergebnisse:

Die Versuchsergebnisse werden in Fig. 13 dargestellt. Während bei Zugabe von normalem Serum-freien Medium die Keratinozyten in die gesetzte Wunde einwandern (Zeile: Medium), kommt es bei den mit rekombinantem YB-1 behandelten Zellen zu keiner signifikanten Einwanderung der Zellen (Zeile: rYB-1 (50 ng)). Wird die in Bakterien exprimierte YB-1 Probe über 10 Minuten bei 95°C erhitzt und anschließend den Keratinozytenkulturen zugegeben, ist ein Einwandern der Zellen zu beobachten, was ausschließt, dass der Inhibierungseffekt durch Lipopolysaccharide hervorgerufen wird (Zeile: inakt. RYB-1 (100 ng)).

Die Zugabe von Anti-YB-1 (Zeile: anti-YB-1 AB4 ("Deutschland") (2) (1 µg) und anti-YB-1 (3) (5 µg)) hat einen ähnlichen Effekt auf die Migration der Keratinozyten, während ein Kontroll-IgG Antikörper die Migration nicht beeinflusst (Zeile: IgG1 (1) (1 µg) und IgG1 (2) (5 µg)). Dies weist darauf hin, dass der verwendete monoklonale Antikörper keine neutralisierende Wirkung auf den YB-1 Effekt hat, sondern diesen eher verstärkt.

### Ausführungsbeispiel 9

### Nachweis von YB-1 Fragmenten mittels monoklonaler Antikörper

In diesem Versuch sind "spezifische" YB-1 Fragmente im Serum von Tumorpatienten und im Urin von Patienten mit IgA-Nephritis mittels monoklonaler Antikörper nachgewiesen worden (Fig. 10B). Mit dem generierten monoklonalen Antikörper "Italien" (AB8, siehe Tabelle 1) wurden das Serum eines Tumorpatienten mit metastasiertem Rektum-Karzinom (TM20) sowie Urin von einem Patienten mit progredienter IgA-Nephritis (K) untersucht. Hierbei fallen neben hochmolekularen Banden um 200 kDa auch Banden mit relativen molekularen Gewichten von 52 und 28 kDa im nicht denaturierenden Probenpuffer (ohne Mercaptoethanol und EDTA) auf. Unter denaturierenden Bedingungen (mit Mercaptoethanol und EDTA) sind die zuvor mit verschiedenen polyklonalen Anti-YB-1 Antikörpern gesehenen Banden mit den relativen Größen von 16 kDa im Serum bei Tumorerkrankung bzw. 23 kDa bei progredienter IgA-Nephritis im Urin nachweisbar. Mit einem Kontrollantikörper ohne spezifische YB-1-Bindung ("Großbritannien", AB9, siehe Tabelle unten) sowie mit dem Anti-Maus Zweit-Antikörper (AB5) alleine sind die Banden bei 16 und 23 kDa nicht nachweisbar.

### Ausführungsbeispiel 10

### Nachweis einer direkten Interaktion von GFP-YB-1 mit HA-getaggtem Notch

Hämagglutinin (HA)-markiertes NOTCH3(EGF) (d.h. die extrazelluläre EGF-haltige Domäne des NOTCH-3 Rezeptors) und GFP bzw. YB-1-GFP werden in HEK293T-Zellen koexprimiert. Die Zelllysate werden mit einem anti-HA-Antikörper immunpräzipitiert, mittels SDS-PAGE aufgetrennt und mit dem Anti-HA-Antikörper detektiert (Spuren 1-3). Das exprimierte Protein N3(EGF) lässt sich in dieser Weise darstellen (Spuren 2 und 3). Weiterhin werden die Zelllysate jeweils mit einem Anti-GFP bzw. dem Anti-HA-Antikörper immunpräzipitiert und anschließend mit Hilfe des Anti-GFP Antikörpers dargestellt. In Spur 9 ist ko-immunpräzipitiertes YB-1-GFP zu sehen. Entsprechende Kontrollversuche, bei denen jeweils nur eines der beiden Proteine exprimiert worden ist, sind negativ (Spuren 5 und 7). I.P. steht für Immunpräzipitation, Blot für die Detektion im Western Blot nach Separation der Proteine.

## Patentansprüche

1. Ein *in vitro* Verfahren zur Bestimmung eines entzündlichen Vorganges oder einer malignen Erkrankung in einem Säugetier, wobei die Bestimmung des entzündlichen Vorganges oder der malignen Erkrankung umfasst:
- Untersuchung einer Probe einer extrazellulären Flüssigkeit des Säugetiers auf das Vorhandensein des YB-1 Proteins und/oder mindestens eines seiner Fragmente in der Probe.

2. Verfahren nach Anspruch 1, das bei Vorhandenseins des YB-1 Proteins und/oder mindestens eines seiner Fragmente in der Probe zusätzlich umfasst:
- Bestimmen des Sekretionsmusters des YB-1 Proteins und/oder seiner Fragmente in der Probe,
insbesondere wobei das Bestimmen des Sekretionsmusters unter Verwendung von HPLC oder eines Western-Blots durchgeführt wird.

3. Verfahren nach einem der Ansprüche 1 oder 2, das zusätzlich umfasst:
- Korrelieren des bestimmten Sekretionsmusters mit anhand von Eichreihen für entzündlichen Vorgänge oder maligne Erkrankungen erstellten Sekretionsmustern.

4. Verfahren nach einem der Ansprüche 1 bis 3, wobei man die Probe mittels Antikörper auf das Vorhandensein des YB-1 Proteins und/oder mindestens eines seiner Fragmente untersucht,
wobei man als Antikörper polyklonale Antikörper verwendet oder
wobei man als Antikörper monoklonale Antikörper verwendet.

5. Verfahren nach einem der vorhergehenden Ansprüche, wobei als Säugetier der Mensch ausgewählt wird.

6. Verfahren nach einem der vorhergehenden Ansprüche, wobei die Körperflüssigkeit ausgewählt wird aus der Gruppe, die Blut, Urin, Lymphe, Plasma, Serum, Schweiss, Nasensekret, Vaginalsekret, Wundexsudat, Sputum, Eiter, Samenflüssigkeit, Stuhl oder Liquor umfasst.

7. Verfahren nach einem der vorhergehenden Ansprüche, wobei der entzündliche Vorgang eine Abstossungsreaktion des Säugetiers nach einer Transplantation oder eine entzündliche Erkrankung sein kann, oder
wobei die maligne Erkrankung aus der Gruppe ausgewählt wird, die aus soliden Tumoren wie einem Pankreas-Karzinom, RektumKarzinom, Magen-Karzinom, Colon-Karzinom, Mamma-Karzinom, Malignes Melanom, Nierenzeil-Karzinom, Larynx-Karzinom, Ovarial-Karzinom, Prostata-Karzinom und einem BronchialKarzinom besteht,
oder
wobei die maligne Erkrankung aus der Gruppe ausgewählt wird, die aus Leukämien wie akuter myeloischer, akuter lymphozytärer, chronisch myeloischer und chronisch lymphatischer Leukämie besteht,
oder
wobei die entzündliche Erkrankung aus der Gruppe ausgewählt wird, die aus nephrologischen Erkrankungen, Asthma, chronisch obstruktiven Lungenerkrankungen, Intimahyperplasie, rheumatoider Arthritis, Atherosklerose, Vaskulitis, Diabetes mellitus, Sepsis, Pankreatitis, Multipler Sklerose, Psoriasis, Colitis und Dermatitis besteht,
und
wobei die nephrologische Erkrankung aus der Gruppe ausgewählt wird, die aus Glomerulonephritis, interstitieller Nephritis, Pyelonephritis, lupoider Nephritis, radiogener Nephritis, vaskulärer Nephropathie und Zystennieren besteht.

8. Verwendung von YB-1 oder mindestens eines Fragmentes davon oder deren Kombination als Marker für die in vitro Diagnose eines entzündlichen Vorganges oder einer malignen Erkrankung in einer extrazellulären Flüssigkeit bei einem Säugetier.

## Claims

1. An in vitro process for the determination of an inflammatory process or a malignant disease in a mammal, wherein the determination of said inflammatory process or malignant disease comprises:
- the examination of a sample of an extracellular fluid from said mammal for the presence of YB-1 protein and/or at least one of its fragments in said sample.

2. The process according to claim 1, which, when YB-1 protein and/or at least one of its fragments is present in said sample, additionally comprises:
- determination of the secretion pattern of said YB-1 protein and/or its fragments in said sample;
in particular, wherein said determination of the secretion pattern is effected by using HPLC or Western blotting.

3. The process according to either of claims 1 or 2, additionally comprising:
- correlating the determined secretion pattern with secretion patterns established from calibration series for inflammatory processes or malignant diseases.

4. The process according to any of claims 1 to 3, wherein the sample is examined for the presence of YB-1 protein and/or at least one of its fragments by means of antibodies;
wherein polyclonal antibodies are used as said antibodies; or
wherein monoclonal antibodies are used as said antibodies.

5. The process according to any of the preceding claims, wherein a human is selected as said mammal.

6. The process according to any of the preceding claims, wherein said body fluid is selected from the group comprising blood, urine, lymph, plasma, serum, sweat, nasal secretion, vaginal secretion, wound exudate, sputum, pus, semen, stool or cerebrospinal fluid.

7. The process according to any of the preceding claims, wherein said inflammatory process is an rejection reaction of said mammal after a transplantation or an inflammatory disease; or
wherein said malignant disease is selected from the group consisting of solid tumors, such as pancreatic carcinoma, rectal carcinoma, gastric carcinoma, colon carcinoma, mammary carcinoma, malignant melanoma, kidney cell carcinoma, laryngeal carcinoma, ovarian carcinoma, prostatic carcinoma and a bronchial carcinoma; or
wherein said malignant disease is selected from the group consisting of leukemias, such as acute myeloic, acute lymphocytic, chronic myeloic and chronic lymphatic leukemias; or
wherein said inflammatory disease is selected from the group consisting of nephrological diseases, asthma, chronic obstructive lung diseases, intimal hyperplasia, rheumatoid arthritis, atherosclerosis, vasculitis, diabetes mellitus, sepsis, pancreatitis, multiple sclerosis, psoriasis, colitis and dermatitis; and
wherein said nephrological disease is selected from the group consisting of glomerulonephritis, interstitial nephritis, pyelonephritis, lupoid nephritis, radiogenic nephritis, vascular nephropathy and cystic kidneys.

8. Use of YB-1 or at least one of its fragments or a combination thereof as a marker for the in-vitro diagnosis of an inflammatory process or a malignant disease in an extracellular fluid in a mammal.

## Revendications

1. Procédé *in vitro* pour déterminer un processus inflammatoire ou une affection maligne chez un mammifère, la détermination du processus inflammatoire ou de l'affection maligne comprenant :
- l'examen d'un échantillon d'un liquide extracellulaire du mammifère pour rechercher la présence de la protéine YB-1 et/ou d'au moins un de ses fragments dans l'échantillon.

2. Procédé selon la revendication 1 qui, en présence de la protéine YB-1 et/ou d'au moins un de ses fragments dans l'échantillon, comprenant en outre :
- la détermination du modèle de sécrétion de la protéine YB-1 et/ou de ses fragments dans l'échantillon,
la détermination du modèle de sécrétion s'effectuant en particulier en utilisant la HPLC ou un transfert western.

3. Procédé selon une des revendications 1 ou 2, qui comprenant en outre :
- la corrélation du modèle de sécrétion déterminé avec des modèles de sécrétion établis à l'aide de critères d'étalonnage pour des processus inflammatoires ou des affections malignes.

4. Procédé selon une des revendications 1 à 3, l'échantillon est examiné au moyen d'anticorps à la recherche de la présence de la protéine YB-1 et/ou d'au moins un de ses fragments,
dans lequel des anticorps polyclonaux sont utilisés en tant qu'anticorps, ou
dans lequel des anticorps monoclonaux sont utilisés en tant qu'anticorps.

5. Procédé selon une des revendications précédentes, dans lequel l'être humain est sélectionné en tant que mammifère.

6. Procédé selon une des revendications précédentes, dans lequel le liquide corporel est sélectionné parmi le groupe consistant en : le sang, l'urine, la lymphe, le plasma, le sérum, la sueur, les sécrétions nasales, les sécrétions vaginales, l'exsudat de plaie, les crachats, le pus, le sperme, les selles ou le liquide céphalo-rachidien.

7. Procédé selon une des revendications précédentes, dans lequel le processus inflammatoire peut être une réaction de rejet du mammifère après une transplantation ou une affection inflammatoire,
ou
dans lequel l'affection maligne est sélectionnée dans le groupe consistant en : les tumeurs solides comme un cancer du pancréas, un cancer du rectum, un cancer de l'estomac, un cancer du côlon, un cancer du sein, un mélanome malin, un cancer des cellules rénales, un cancer du larynx, un cancer ovarien, un cancer de la prostate et un carcinome bronchial,
ou
dans lequel l'affection maligne est sélectionnée dans le groupe consistant en : les leucémies comme la leucémie myéloïde aiguë, la leucémie lymphocytaire aiguë, la leucémie myéloïde chronique et la leucémie lymphatique chronique,
ou
dans lequel l'affection inflammatoire est sélectionnée dans le groupe consistant en : les affections néphrologiques, d'asthme, de maladies pulmonaires obstructives chroniques, d'hyperplasie de l'intima, de polyarthrite rhumatoïde, d'athérosclérose, de vascularite, de diabète sucré, de septicémie, de pancréatite, de sclérose en plaques, de psoriasis, de colite et de dermatite,
et
dans lequel l'affection néphrologique est sélectionnée dans le groupe consistant en : la glomérulonéphrite, de la néphrite interstitielle, de la pyélonéphrite, de la néphrite lupique, de la néphrite radique, de la néphropathie vasculaire et de la polykystose rénale.

8. Utilisation de YB-1 ou d'au moins un fragment de celle-ci ou utilisation de leur combinaison en tant que marqueur pour le diagnostic *in vitro* d'un processus inflammatoire ou d'une affection maligne dans un liquide extracellulaire chez un mammifère.
